# EUROPÄISCHE PATENTSCHRIFT

(11) **EP 0 805 805 B1**
(45) Veröffentlichungstag und Bekanntmachung des Hinweises auf die Patenterteilung: **04.04.2001**
(21) Anmeldenummer: 96900930.7
(22) Anmeldetag: 10.01.1996
(51) Int. Cl.: C07D 273/02, A01N 43/88, C07D 413/12, C07D 417/12

(54) **SUBSTITUIERTE ARYLAZADIOXACYCLOALKENE FUNGIZIDE**
SUBSTITUTED ARYLAZADIOXACYCLO ALKENE FUNGICIDES
ARYLAZADIOXACYCLOALCENES SUBSTITUES FONGICIDES

(30) Priorität: 23.01.1995 DE 19501842
(43) Veröffentlichungstag der Anmeldung: 12.11.1997
(73) Patentinhaber: BAYER AG, 51368 Leverkusen (DE)
(72) Erfinder: GERDES, Peter, D-52080 Aachen (DE); GAYER, Herbert, D-40789 Monheim (DE); KUHNT, Dietmar, D-51399 Burscheid (DE); TIEMANN, Ralf, D-51375 Leverkusen (DE); STENZEL, Klaus, D-40595 Düsseldorf (DE)
(86) Internationale Anmeldenummer: EP9600069
(87) Internationale Veröffentlichungsnummer: WO9622983

(56) Entgegenhaltungen:
- PATENT ABSTRACTS OF JAPAN vol. 14 no. 544 (C-0784) ,4.Dezember 1990 & JP,A,02 233602 (HOKKO CHEM IND CO LTD) 19.September 1990,
- PATENT ABSTRACTS OF JAPAN vol. 14 no. 126 (C-0699) ,9.März 1990 & JP,A,02 001484 (HOKKO CHEM IND CO LTD) 5.Januar 1990, in der Anmeldung erwähnt
- PATENT ABSTRACTS OF JAPAN vol. 13 no. 536 (C-660) ,29.November 1989 & JP,A,01 221371 (HOKKO CHEM IND CO LTD) 4.September 1989,

## Beschreibung

Die Erfindung betrifft neue substituierte Arylazadioxacycloalkene, mehrere Verfahren zu ihrer Herstellung und ihre Verwendung als Fungizide.

Es ist bekannt, daß bestimmte substituierte Arylazadioxacycloalkene fungizide Eigenschaften aufweisen [vgl. Hokko Chemical Industry, JP-A 02001484; zitiert in Chem. Abstr. 113:6381 (1990)].

Die Wirkung dieser Verbindungen ist jedoch in vielen Fällen unbefriedigend.

Es wurden nun die neuen substituierten Arylazadioxacycloalkene der allgemeinen Formel (I) gefunden, 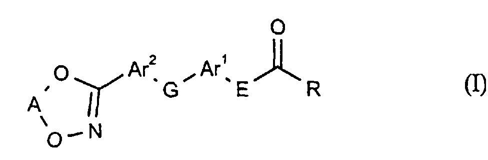 in welcher
- A: für -C₂ H₄- steht,
- Ar¹: für jeweils gegebenenfalls substituiertes Phenylen oder Naphthylen, für mono-oder bicyclisches Heteroarylen mit jeweils 5 oder 6 Ringgliedem oder für benzokondensiertes Heteroarylen mit 5 oder 6 Ringgliedern, von denen jeweils mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
- Ar²: für jeweils gegebenenfalls einfach oder mehrfach, gleich oder verschieden substituiertes Phenylen, Naphthylen oder für Heteroarylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
- E: für eine der nachstehenden Gruppierungen steht: 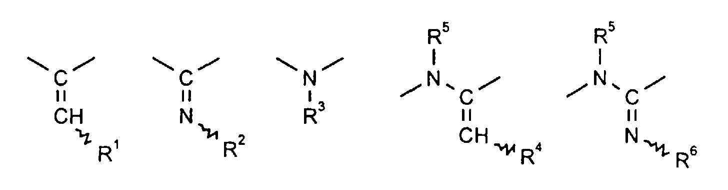 worin
R¹ für Wasserstoff, Halogen, Cyano oder für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylresten steht,
R² für Wasserstoff, Amino, Cyano oder für jeweils gegebenenfalls durch Halogen, Cyano oder C₁-C₄-Alkoxy substituiertes Alkyl, Alkoxy, Alkylamino oder Dialkylamino mit jeweils 1 bis 6 Kohlenstoffatomen in den Alkylresten steht,
R³ für Wasserstoff, Cyano oder für jeweils gegebenenfalls durch Halogen, Cyano oder C₁-C₄-Alkoxy substituiertes Alkyl, Alkenyl oder Alkinyl mit jeweils bis zu 6 Kohlenstoffatomen oder für jeweils gegebenenfalls durch Halogen, Cyano, Carboxy, C₁-C₄-Alkyl oder C₁-C₄-Alkoxycarbonyl substituiertes Cycloalkyl oder Cycloalkylalkyl mit 3 bis 6 Kohlenstoffatomen in den Cycloalkylteilen und gegebenenfalls 1 bis 4 Kohlenstoffatomen im Alkylteil steht,
R⁴ für Wasserstoff, Halogen, Cyano oder für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylresten steht,
R⁵ für Alkyl mit 1 bis 6 Kohlenstoffatomen steht und
R⁶ für Wasserstoff, Amino, Cyano oder für jeweils gegebenenfalls durch Halogen, Cyano oder C₁-C₄-Alkoxy substituiertes Alkyl, Alkoxy, Alkylamino oder Dialkylamino mit jeweils 1 bis 6 Kohlenstoffatomen in den Alkylresten steht;
- G: für eine Einfachbindung, für Sauerstoff, Schwefel oder für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁷)=N-O-, -C(R⁷)=N-O-CH₂-, -N(R⁸)-, -CQ-N(R⁸)-, -N(R⁸)-CQ-, -Q-CQ-N(R⁸)-, -N=C(R⁷)-Q-CH₂-, -CH₂-O-N=C(R⁷)-, -N(R⁸)-CQ-Q-, -CQ-N(R⁸)-CQ-Q-, -N(R⁸)-CQ-Q-CH₂-, -Q-C(R⁷)=N-O-CH₂-, -N(R⁸)-C(R⁷)=N-O-CH₂-, -O-CH₂-C(R⁷)=N-O-, -N=N-C(R⁷)=N-O-, -T-Ar³- oder -T-Ar³-Q- steht, wobei
n für die Zahlen 0, 1 oder 2 steht,
Q für Sauerstoff oder Schwefel steht,
R⁷ für Wasserstoff, Cyano, für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
R⁸ für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C1-C4-Alkoxy-carbonyl substituiertes Cycloalkyl mit 3 bis 6 Kohlenstoffatomen steht und
Ar³ für jeweils gegebenenfalls einfach oder mehrfach, gleich oder verschieden substituiertes Phenylen, Naphthylen, Cycloalkylen oder für Heteroarylen oder Heterocycloalkylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, sowie;
Cycloalkyl mit 3 bis 6 Kohlenstoffatomen und

T für eine Einfachbindung, für Sauerstoff, Schwefel, -CH₂-O-, CH₂-S-oder für Alkandiyl mit 1 bis 3 Kohlenstoffatomen steht,
- R: für Alkyl, Alkoxy, Alkylamino, Hydroxylamino, Alkoxyamino oder Dialkylamino mit jeweils 1 bis 4 Kohlenstoffatomen steht.

Weiterhin wurde gefunden, daß man die neuen substituierten Arylazadioxacycloalkene der allgemeinen Formel (I) erhält, wenn man
a) Hydroxyverbindungen der allgemeinen Formel (II) 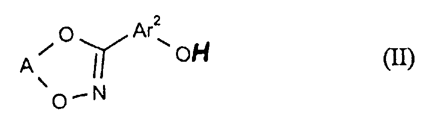 in welcher
   - A und Ar²: die oben angegebene Bedeutung haben,
   mit Halogenverbindungen der allgemeinen Formel (III) 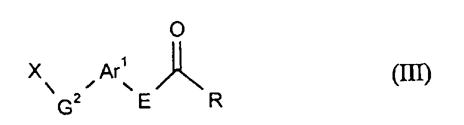 in welcher
   - G²: für -Ar³-Q- oder für -Ar³- steht,
   - Ar¹, Ar³, E, Q und R: die oben angegebene Bedeutung haben und
   - X: für Halogen steht,
   gegebenenfalls in Gegenwart eines Säureakzeptors und gegebenenfalls in Gegenwart eines Verdünnungsmittels umsetzt;
b) Hydroxyverbindungen der allgemeinen Formel (II) 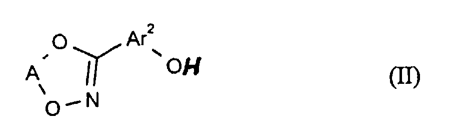 in welcher
   - A und Ar²: die oben angegebene Bedeutung haben,
   mit Halogenverbindungen der allgemeinen Formel (IV) 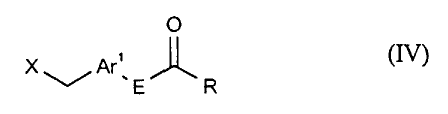 in welcher
   - Ar¹, E, und R: die oben angegebene Bedeutung haben und
   - X: für Halogen steht,
   gegebenenfalls in Gegenwart eines Säureakzeptors und gegebenenfalls in Gegenwart eines Verdünnungsmittels umsetzt;
c) Ester der allgemeinen Formel (I-1) 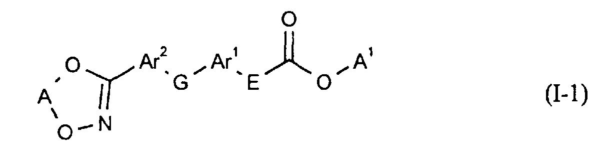 in welcher
   - A, Ar¹, Ar², E und G: die oben angegebene Bedeutung haben und
   - A¹: für Alkyl steht,
   mit Aminen der allgemeinen Formel (V), 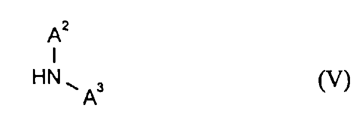 in welcher
   - A² und A³: unabhängig voneinander jeweils für Wasserstoff, jeweils gegebenenfalls substituiertes Alkyl, Alkoxy oder Hydroxy stehen,
- oder mit einem Säureadditionskomplex hiervon -
gegebenenfalls in Gegenwart eines Säureakzeptors und gegebenenfalls in Gegenwart eines Verdünnungsmittels umsetzt.

Schließlich wurde gefunden, daß die neuen substituierten Carbonsäureamide der allgemeinen Formel (I) sehr starke fungizide Wirkung zeigen.

Die erfindungsgemäßen Verbindungen können gegebenenfalls als Mischungen verschiedener möglicher isomerer Formen, insbesondere von Stereoisomeren, wie z. B. E-und Z-Isomeren vorliegen. Es werden sowohl die E- als auch die Z-Isomeren, wie auch beliebige Mischungen dieser Isomeren beansprucht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- A: für 1,2-Ethylen steht,
- Ar¹: für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Cyclopropyl, Trifluormethyl, Methoxy, Ethoxy, Methylthio, Methylsulfinyl oder Methylsulfonyl,
- Ar²: für jeweils gegebenenfalls einfach bis dreifach, gleich oder verschieden substituiertes Phenylen, Naphthylen, Furandiyl, Thiophendiyl, Oxazoldiyl, Isoxazoldiyl, Thiazoldiyl, Isothiazoldiyl, 1,2,4-Oxadiazoldiyl, 1,3,4-Oxadiazoldiyl, 1,2,4-Thiadiazoldiyl, 1,3,4-Thiadiazoldiyl, Pyridindiyl, Pyrimidindiyl, Pyridazindiyl, Pyrazindiyl, 1,2,3-Triazindiyl, 1,2,4-Triazindiyl, 1,3,5-Triazindiyl, steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Cyclopropyl, Methoxy, Ethoxy, n- oder i-Propoxy, Methylthio, Ethylthio, n-oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
- E: für eine der nachstehenden Gruppierungen steht: 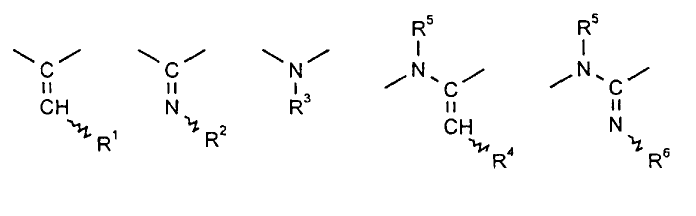 worin
R¹ für Wasserstoff, Fluor, Chlor, Brom, Cyano oder für jeweils gegebenenfalls durch Fluor, Chlor, Cyano, Methoxy oder Ethoxy substituiertes Methyl, Ethyl, Propyl, Methoxy, Ethoxy, Methylthio, Ethylthio, Methylamino, Ethylamino oder Dimethylamino steht,
R² für Wasserstoff, Amino, Cyano oder für jeweils gegebenenfalls durch Fluor, Chlor, Cyano, Methoxy oder Ethoxy substituiertes Methyl, Ethyl, Methoxy, Ethoxy, Methylamino, Ethylamino oder Dimethylamino steht,
R³ für Wasserstoff, Cyano oder für jeweils gegebenenfalls durch Fluor, Cyano, Methoxy oder Ethoxy substituiertes Methyl, Ethyl n- oder i-Propyl, n-, i- oder s-Butyl, für Allyl oder Propargyl oder für jeweils gegebenenfalls durch Fluor, Chlor, Cyano, Carboxy, Methyl, Ethyl, n-oder i-Propyl, Methoxy-carbonyl oder Ethoxy-carbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R⁴ für Wasserstoff, Fluor, Chlor, Brom, Cyano oder für jeweils gegebenenfalls durch Fluor, Chlor, Cyano, Methoxy oder Ethoxy substituiertes Methyl, Ethyl, Propyl, Methoxy, Ethoxy, Methylthio, Ethylthio, Methylamino, Ethylamino oder Dimethylamino steht,
R⁵ für Methyl, Ethyl n- oder i-Propyl, n-, i- oder s-Butyl steht,
R⁶ für Wasserstoff, Amino, Cyano oder für jeweils gegebenenfalls durch Fluor, Chlor, Cyano, Methoxy oder Ethoxy substituiertes Methyl, Ethyl, Methoxy, Ethoxy, Methylamino, Ethylamino oder Dimethylamino steht, und
G für eine Einfachbindung, für Sauerstoff, Schwefel oder für jeweils gegebenenfalls durch Fluor, Chlor, Brom, Hydroxy, Methyl, Ethyl, n-oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁷)=N-O-, -C(R⁷)=N-O-CH₂-, -N(R⁸)-, -CQ-N(R⁸)-, - N(R⁸)-CQ-, -Q-CQ-N(R⁸)-, -N=C(R⁷)-Q-CH₂-, -CH₂-O-N=C(R⁷)-, -N(R⁸)-CQ-Q-, -CQ-N(R⁸)-CQ-Q-, -N(R⁸)-CQ-Q-CH₂-, -Q-C(R⁷)=N-O-CH₂-, -N(R⁸)-C(R⁷)=N-O-CH₂-, -O-CH₂-C(R⁷)=N-O-, -N=N-C(R⁷)=N-O-, -T-Ar³- oder -T-Ar³-Q- steht, wobei
n für die Zahlen 0, 1 oder 2 steht,
Q für Sauerstoff oder Schwefel steht,
R⁷ für Wasserstoff, Cyano, für jeweils gegebenenfalls durch Fluor, Chlor, Cyano, Methoxy oder Ethoxy substituiertes Methyl, Ethyl, n- oder i-Propyl, n-, i- oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und
R⁸ für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für jeweils gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht,
Ar³ für jeweils gegebenenfalls einfach bis dreifach substituiertes Phenylen, Naphthylen, Furandiyl, Thiophendiyl, Oxazoldiyl, Isoxazoldiyl, Thiazoldiyl, Isothiazoldiyl, 1,2,4-Oxadiazoldiyl, 1,3,4-Oxadiazoldiyl, 1,2,4-Thiadiazoldiyl, 1,3,4-Thiadiazoldiyl, Pyridindiyl, Pyrimidindiyl, Pyridazindiyl, Pyrazindiyl, 1,2,3-Triazindiyl, 1,2,4-Triazindiyl, 1,3,5-Triazindiyl, Oxirandiyl, Oxetandiyl, Tetrahydrofurandiyl, Perhydropyrandiyl oder Pyrrolidindiyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, , Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl oder Cyclopropyl und
T für eine Einfachbindung, für Sauerstoff, Schwefel, -CH₂-O-, CH₂-S-, Methylen, Ethylen oder Propylen steht und
- R: für Methyl, Ethyl, n- oder i-Propyl, Methoxy, Ethoxy, n- oder i-Propoxy, Methylamino, Ethylamino, n- oder i-Propylamino, Hydroxylamino, Methoxyamino, Dimethylamino, Diethylamino steht.

Ganz besonders bevorzugt sind Verbindungen der allgemeinen Formel (I),
in welcher
- A: für 1,2-Ethylen steht,
- Ar¹: für ortho-Phenylen, Pyridin-2,3-diyl oder Thiophen-2,3-diyl steht,
- Ar²: für jeweils gegebenenfalls einfach bis dreifach, gleich oder verschieden substituiertes Phenylen steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluomethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy,
- E: für eine der nachstehenden Gruppierungen steht 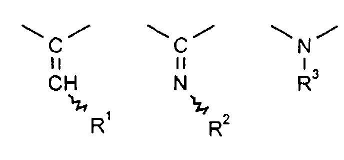 worin
R¹ und R² jeweils für Methoxy stehen und
R³ für Wasserstoff, Cyano, Methyl, Ethyl n- oder i-Propyl, Methoxy oder Ethoxy steht,
G für Sauerstoff oder für jeweils gegebenenfalls durch Fluor, Chlor oder Brom substituiertes Dimethylen (Ethan-1,2-diyl), Ethen-1,2-diyl oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁷)=N-O-, -C(R⁷)=N-O-CH₂-, -N(R⁸)-, -CQ-N(R⁸)-, -N(R⁸)-CQ-, -Q-CQ-N(R⁸)-, -N=C(R⁷)-Q-CH₂-, -CH₂-O-N=C(R⁷)-, -N(R⁸)-CQ-Q-, -CQ-N(R⁸)-CQ-Q-, -N(R⁸)-CQ-Q-CH₂-, -Q-C(R⁷)=N-O-CH₂-, -N(R⁸)-C(R⁷)=N-O-CH₂-, -O-CH₂-C(R⁷)=N-O-, -N=N-C(R⁷)=N-O-, -T-Ar³- oder-T-Ar³-Q- steht, wobei
n für die Zahlen 0, 1 oder 2 steht,
Q für Sauerstoff oder Schwefel steht,
R⁷ für Wasserstoff, Cyano, Methyl, Ethyl oder Cyclopropyl steht und
R⁸ für Wasserstoff, Methyl, Ethyl oder Cyclopropyl steht,
Ar³ für jeweils gegebenenfalls einfach bis dreifach, gleich oder verschieden substituiertes Phenylen, 1,2,4-Thiadiazoldiyl, 1,3,4-Thiadiazoldiyl, 1,2,4-Oxadiazoldiyl, 1,3,4-Oxadiazoldiyl, Pyridindiyl, Pyrimidindiyl, Pyridazindiyl, Pyrazindiyl, 1,2,3-Triazindiyl, 1,2,4-Triazindiyl oder 1,3,5-Triazindiyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, Cyclopropyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl und
T für eine Einfachbindung, für Sauerstoff, Schwefel, -CH₂-O-, CH₂-S-, Methylen, Ethylen oder Propylen steht und
- R: für Methyl, Ethyl, Methoxy, Methylamino oder Hydroxylamino steht.

Eine besonders bevorzugte Gruppe erfindungsgemäßer Verbindungen sind diejenigen Verbindungen der Formel (I),
in welcher
- A: für 1,2-Ethylen steht,
- Ar¹: für ortho-Phenylen, Pyridin-2,3-diyl oder Thiophen-2,3-diyl steht,
- Ar²: für jeweils gegebenenfalls einfach bis dreifach, gleich oder verschieden substituiertes Phenylen steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluomethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy,
- E: für eine der nachstehenden Gruppierungen steht, 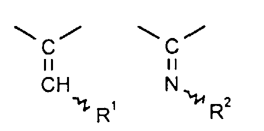 worin
- R¹ und R²: jeweils für Methoxy stehen und
- G: für -O-CH₂ steht und
- R: für Methyl, Ethyl, Methoxy, Methylamino oder Hydroxylamino steht.

Ebenfalls ganz besonders bevorzugt sind Verbindungen der allgemeinen Formel (I),
in welcher
- A: für 1,2-Ethylen steht,
- Ar¹: für ortho-Phenylen, Pyridin-2,3-diyl oder Thiophen-2,3-diyl steht,
- Ar²: für jeweils gegebenenfalls einfach bis dreifach, gleich oder verschieden substituiertes Phenylen steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluomethylthio, Trifluormethylthio, Difluorchlormethylthio, Trifluormethylsulfinyl oder Trifluormethylsulfonyl, Methoxycarbonyl, Ethoxy-carbonyl, Methoximinomethyl, Ethoximinomethyl, Methoximinoethyl, Ethoximinoethyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy,
- E: für eine der nachstehenden Gruppierungen steht 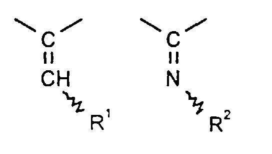 worin
- R¹ und R²: jeweils für Methoxy stehen und
- G: für -C(R⁷)=N-O-CH₂- steht, wobei
R⁷ für Wasserstoff, Cyano, Methyl, Ethyl oder Cyclopropyl steht und
- R: für Methyl, Ethyl, Methoxy, Methylamino oder Hydroxylamino steht.

Weiterhin ganz besonders bevorzugt sind Verbindungen der allgemeinen Formel (I),
in welcher
- A: für 1,2-Ethylen steht,
- Ar¹: für ortho-Phenylen steht,
- Ar²: für jeweils gegebenenfalls einfach bis dreifach, gleich oder verschieden substituiertes Phenylen steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluomethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy,
- E: für eine der nachstehenden Gruppierungen steht: 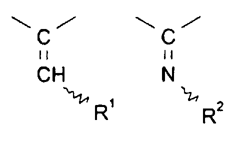 worin
- R¹ und R²: jeweils für Methoxy stehen,
- G: für -T-Ar³-Q- steht, wobei
Q für Sauerstoff oder Schwefel steht,
Ar³ für 1,2,4-Thiadiazoldiyl, 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio, Trifluormethyl, Difluormethoxy, Trifluormethoxy, Difluorchlormethoxy substituiertes, Pyridindiyl, Pyrimidindiyl oder 1,3,5-Triazindiyl steht,
T für eine Einfachbindung, für Sauerstoff, Schwefel, -CH₂-O-, CH₂-S-, Methylen, Ethylen oder Propylen steht und
- R: für Methyl, Ethyl, Methoxy, Methylamino oder Hydroxylamino steht.

Die oben aufgeführten allgemeinen oder in Vorzugsbereichen angegebenen Reste-definitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Beispiele für die erfindungsgemäßen Verbindungen sind in der Tabelle 1 gemäß der allgemeinen Formel Ia aufgeführt:

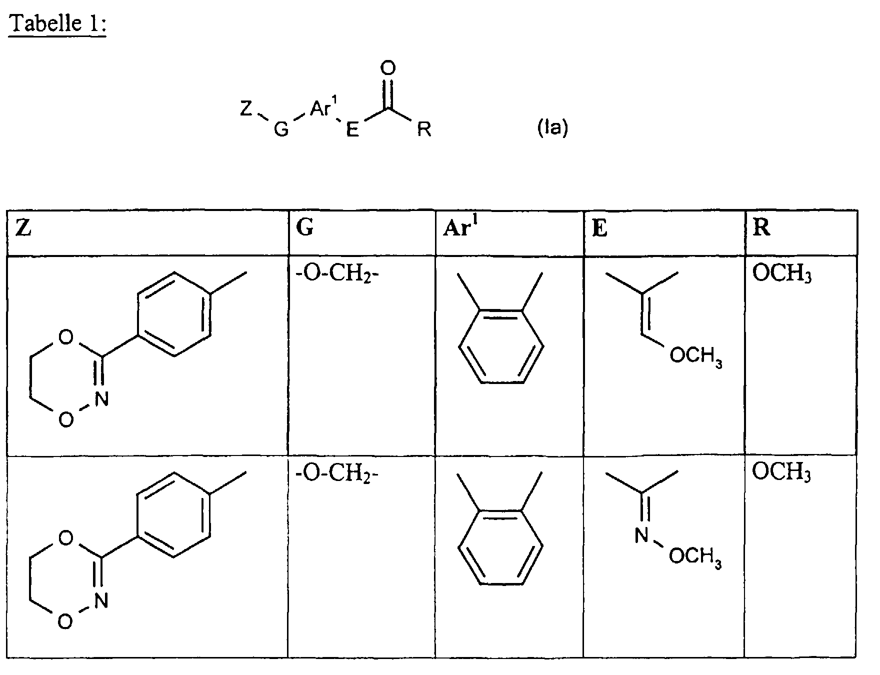

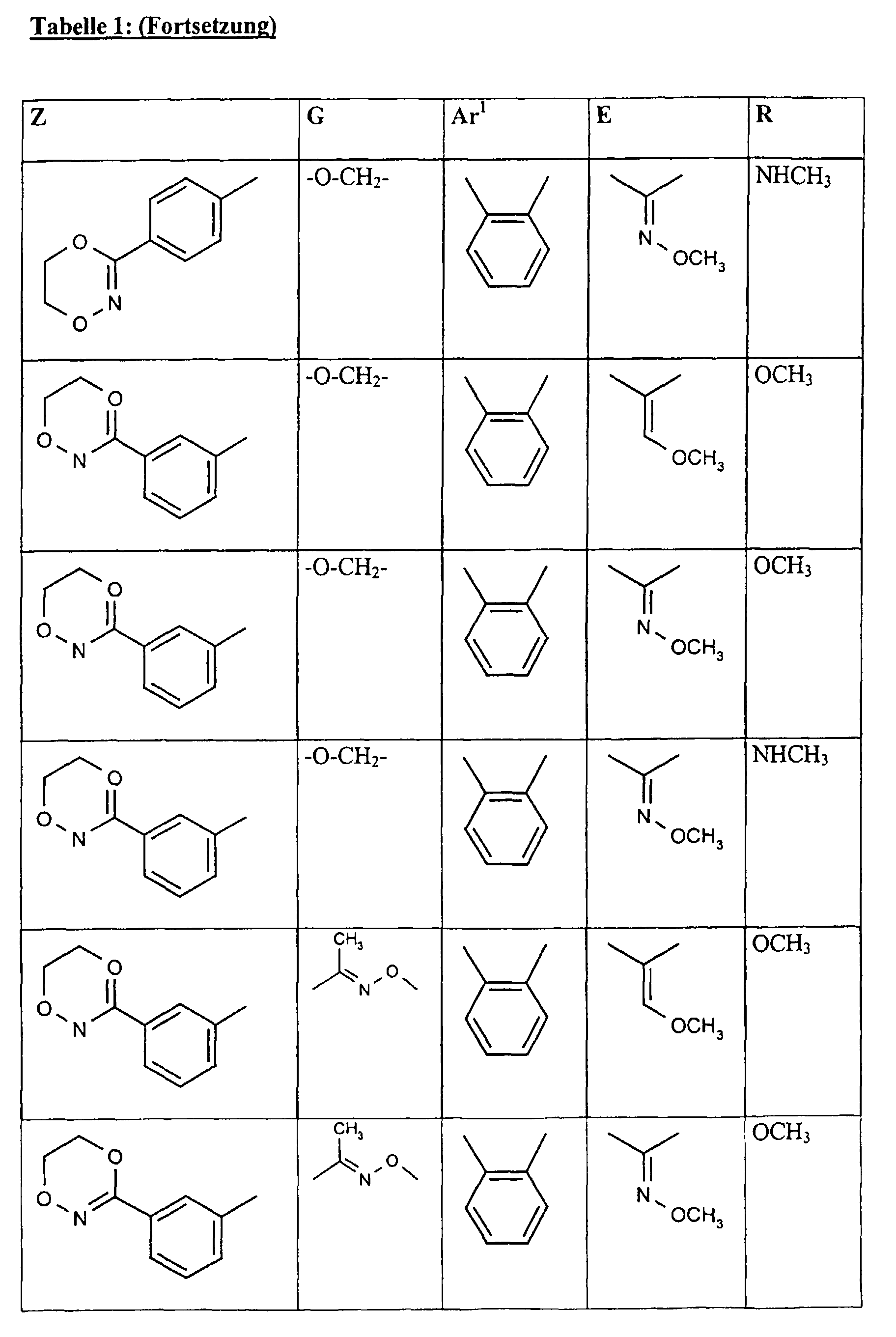

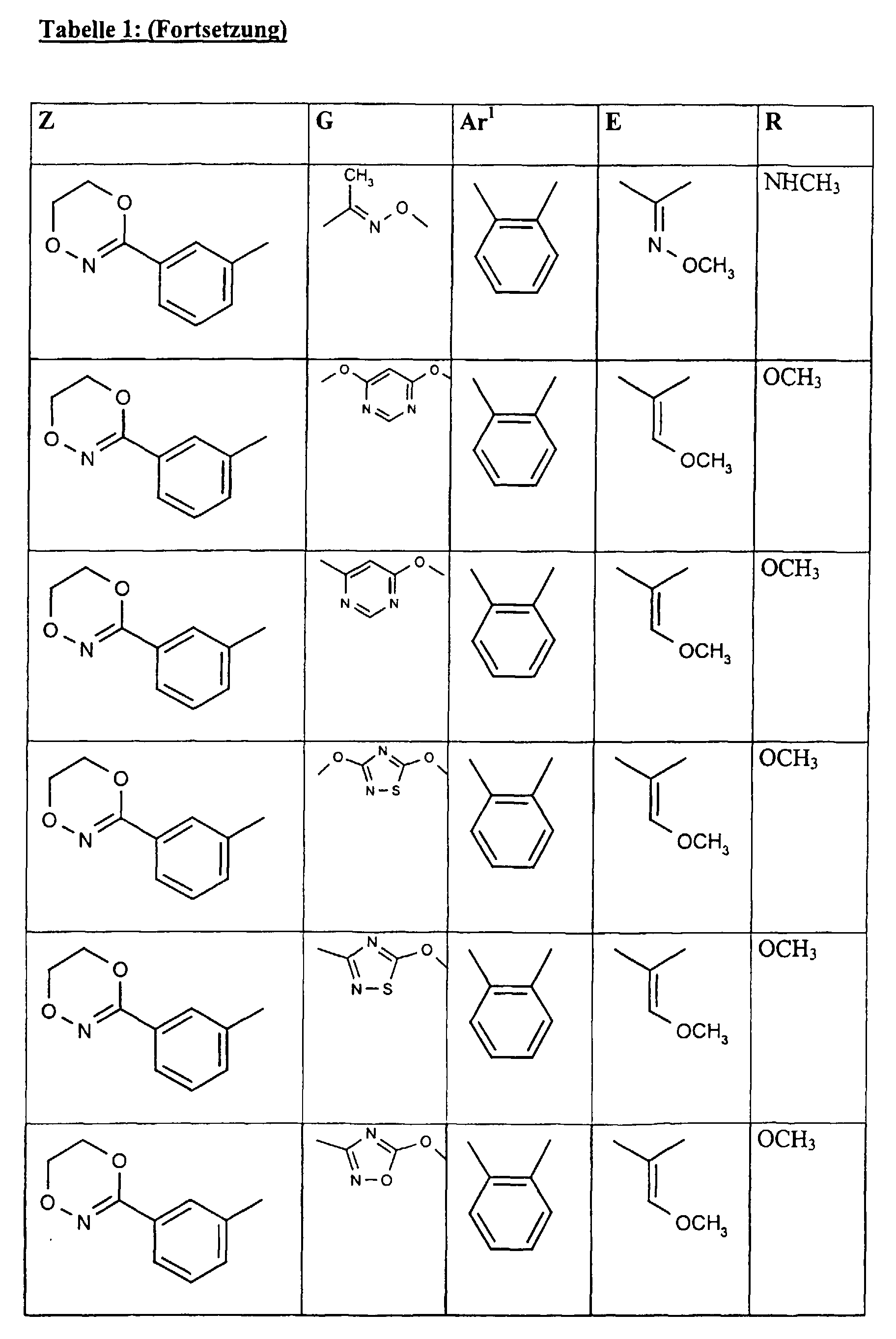

Die zur Durchführung der erfindungsgemäßen Verfahren a) und b) als Ausgangsstoffe benötigten Hydroxyverbindungen sind durch die Formel (II) allgemein definiert. In dieser Formel (II) haben A und Ar² vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für A und Ar² angegeben wurde.

Die Hydroxyverbindungen der Formel (II) sind bekannt und/oder können nach an sich bekannten Verfahren hergestellt werden (vgl. JP-A 02001484).

Die weiterhin zur Durchführung des erfindungsgemäßen Verfahrens a) als Ausgangsstoffe benötigten Halogenverbindungen sind durch die Formel (III) allgemein definiert. In dieser Formel (III) haben Ar¹, Ar³, E, Q und R vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für Ar¹, Ar³, E, Q und R angegeben wurde. X steht für Halogen, bevorzugt für Chlor oder Fluor.

Die Halogenverbindungen der Formel (III) sind bekannt und/oder können nach an sich bekannten Verfahren hergestellt werden (vgl. EP-A 382375).

Die weiterhin zur Durchführung des erfindungsgemäßen Verfahrens b) als Ausgangsstoffe benötigten Halogenverbindungen sind durch die Formel (IV) allgemein definiert. In dieser Formel (IV) haben Ar¹, E und R vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für Ar¹, E und R angegeben wurde. X steht für Halogen, bevorzugt für Chlor oder Brom.

Die Halogenverbindungen der Formel (IV) sind bekannt und/oder können nach an sich bekannten Verfahren hergestellt werden (vgl. EP-A 226917 und EP-A 254426).

Die zur Durchführung des erfindungsgemäßen Verfahrens (c) als Ausgangsstoffe benötigten Ester sind durch die Formel (I-1) allgemein definiert. In dieser Formel (I-1) haben A, Ar¹, Ar², E und G vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für A, Ar¹, Ar², E und G angegeben wurde. A¹ steht für Alkyl, bevorzugt Methyl. Die Verbindungen der Formel (I-1) sind erfindungsgemäße Verbindungen und können nach den erfindungsgemäßen Verfahren (a) und (b) erhalten werden.

Die weiterhin zur Durchführung des erfindungsgemäßen Verfahrens (c) als Ausgangsstoffe benötigten Amine sind durch die Formel (V) allgemein definiert. In der Formel (V) stehen A² und A³ jeweils unabhängig voneinander für Wasserstoff, jeweils gegebenenfalls substituiertes Alkyl, Alkoxy oder Hydroxy, A² steht bevorzugt für Wasserstoff, A³ steht bevorzugt für Methyl oder Hydroxy. Die Amine der Formel (V) oder deren Säureadditionskomplexe sind bekannte Reagenzien in der organischen Chemie.

Die erfindungsgemäßen Verfahren a), b) und c) werden gegebenenfalls in Gegenwart eines Verdünnungsmittels durchgeführt. Als solche kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Sufone, wie Sulfolan. Das erfindungsgemäße Verfahren c) kann gegebenenfalls auch in Alkoholen, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemischen mit Wasser oder in reinem Wasser durchgeführt werden.

Die erfindungsgemäßen Verfahren a), b) und c) werden gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natriummethylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen von -20°C bis +200°C. Verfahren a) und b) werden vorzugsweise bei Temperaturen von 20°C bis 150°C, Verfahren c) wird vorzugsweise bei Temperaturen von 0 - 80°C durchgeführt.

Zur Durchführung der erfindungsgemäßen Verfahren a) und b) zur Herstellung der erfindungsgemäßen Verbindungen der Formel (I) setzt man je Mol an Hydroxyderivat der Formel (II) im allgemeinen 0,5 bis 5 Mol, vorzugsweise 0,8 bis 1,5 Mol an Halogenvervindung der Formel (III) bzw. (IV) ein.

Zur Durchführung des erfindungsgemäßen Verfahrens c) zur Herstellung der erfindungsgemäßen Verbindungen der Formel (I) setzt man pro Mol des Esters der Formel (I-1) im allgemeinen 1 bis 100 Mol, vorzugsweise 1 bis 20 Mol an Amin der Formel (V) ein.

Die Reaktionsdurchführung, Aufarbeitung und Isolierung der Reaktionsprodukte erfolgt nach bekannten Verfahren (vgl. auch die Herstellungsbeispiele).

Die erfindungsgemäßen Wirkstoffe weisen eine starke mikrobizide Wirkung auf und werden zur Bekämpfung von Schädlingen, insbesondere von unerwünschten Mikroorganismen praktisch eingesetzt. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen, erlaubt eine Behandlung von oberirdischen Pflanzenteilen, sowie auch eine Behandlung von Pflanz- und Saatgut und des Bodens.

Dabei werden die erfindungsgemäßen Wirkstoffe mit besonders gutem Erfolg zur Bekämpfung von Krankheiten im Wein-, Obst- und Gemüseanbau, wie beispielsweise gegen Plasmopara- und Podosphaera-Arten eingesetzt.

Die erfindungsgemäßen Wirkstoffe werden auch mit gutem Erfolg zur Bekämpfung von Getreidekrankheiten, wie beispielsweise gegen Septoria-, Pyricularia-, Pyrenophora- sowie Cochliobolus-Arten eingesetzt. Außerdem zeigen die erfindungsgemäßen Wirkstoffe eine besonders gute in vitro Wirkung.

Die Wirkstoffe werden in Abhängigkeit von ihren jeweiligen physikalischen und/oder chemischen Eigenschaften gegebenenfalls in übliche Formulierungen übergeführt, wie z. B. Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es werden in den Formulierungen gegebenenfalls Haftmittel wie Carboxymethylcellulose, natürliche und synthetische, pulverige, körnige oder latexförmige Polymere verwendet, wie z. B. Gummiarabicum, Polyvinylalkohol, Polyvinylacetat, sowie natürliche Phospholipide, wie Kephaline und Lecithine und synthetische Phospholipide. Weitere mögliche Additive sind mineralische und vegetabile Öle.

Gegebenenfalls werden Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink zugesetzt.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen.

In vielen Fällen werden dabei synergistische Wirkungen beobachtet.

Für die Mischungen kommen beispielsweise infrage:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyano-phenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb,
Cymoxanil, Cyproconazole, Cyprofuram,
Dichlorophen, Diclobutrazol, Diclofluanid, Diclomezin, Dicloran, Diethofencarb,
Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfemaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol,
Methasulfocarb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157
419, CGA 184699, Chloethocarb, Chlorethoxyfos, Chloretoxyfos, Chlorfenvinphos,
Chlorfluazuron, Chlormephos, Chlorpyrifos, Chlorpyrifos M, Cis-Resmethrin,
Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb,
Fenoxycarb, Fenpropathrin, Fenpyrad, Fenpyroximat, Fenthion, Fenvalerate,
Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lamda-cyhalothrin, Lufenuron,
Malathion, Mecarbam, Mervinphos, Mesulfenphos, Metaldehyd, Methacrifos,
Methamidophos, Methidathion, Methiocarb, Methomyl, Metolcarb, Milbemectin,
Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet,
Phosphamdon, Phoxim, Pirimicarb, Pirimiphos M, ,Primiphos A, Profenofos,
Profenophos, Promecarb, Propaphos, Propoxur, Prothiofos, Prothiophos, Prothoat,
Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Gegebenenfalls werden die erfindungsgemäßen Wirkstoffe auch mit anderen bekannten Wirkstoffen, wie Herbiziden oder auch mit Düngemitteln und Wachstumsregulatoren gemischt.

Die Wirkstoffe werden als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verstäuben, Verschäumen, Bestreichen usw. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel 1

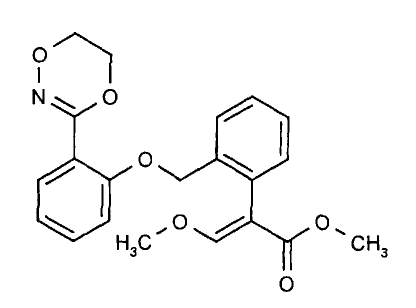

2,85 g (0,01 mol) 2-(2-Brommethylphenyl)-3-methoxyacrylsäuremethylester, 1,8 g (0,01 mol) 3-(2-Hydroxyphenyl)-5,6-dihydro-1,4,2-dioxazin und 1,3 g Kalium-tert-butanolat werden in 50 ml Tetrahydrofuran 4 Stunden lang unter Rückfluß erhitzt. Danach engt man die Lösung bis zur Trockne ein, nimmt in 50 ml Wasser auf und extrahiert mit Essigsäureethylester. Die organische Phase wird getrocknet und unter vermindertem Druck eingeengt. Zum Schluß reinigt man den Rückstand durch Säulenchromatographie an Kieselgel mit Dichlormethan/Essigsäureethylester (10:1).

Man erhält 2,9 g (76,3 %) der Zielverbindung als hellgelbes Öl.
¹H-NMR (CDCl₃/TMS) δ (ppm): 3,71 (s,3H), 3,82 (s,3H), 4,20 (m,2H), 4,47 (m, 2H), 5,04 (s,2H), 6,75 - 7,5 (m,8H), 7,61 (s,1H).

### Beispiel 2

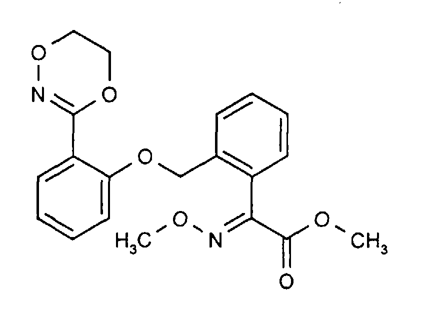

8,6 g (0.03 mol) 2-(2-Brommethylphenyl)-2-methoximinoessigsäuremethylester, 5,4 g (0.03 mol) 3-(2-Hydroxyphenyl)-5,6-dihydro-1,4,2-dioxazin und 1 g (0.033 mol) Natriumhydrid (80%ig) werden in 50 ml Tetrahydrofuran 4 Stunden lang unter Rückfluß erhitzt. Danach engt man die Lösung bis zur Trockne ein, nimmt in 50 ml Wasser auf und extrahiert mit Essigsäureethylester. Die organische Phase wird getrocknet und unter vermindertem Druck eingeengt. Zum Schluß reinigt man den Rückstand durch Säulenchromatographie an Kieselgel mit Dichlormethan/Essigsäureethylester (10:1).

Man erhält 3,5 g (31 %) der Zielverbindung als weißen Feststoff.
Schmelzpunkt : 179 °C

### Beispiel 3

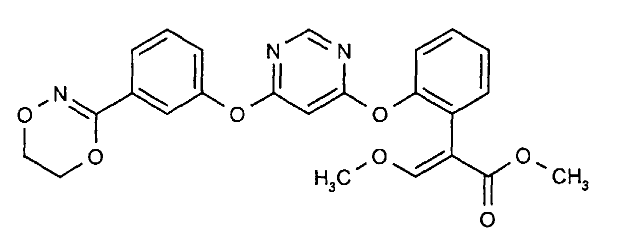

Zu einer Mischung von 6,4 g (0,02 mol) E-3-Methoxy-2-[2-(6-chlor-4-pyrimidinyloxy)-phenyl]-acrylsäuremethylester (vergl. z.B. EP 382375) und 3,6 g (0.02 mol) 3-(3-Hydroxyphenyl)-5,6-dihydro-1,4,2-dioxazin in 50 ml absolutem N,N-Dimethylformarnid gibt man bei Raumtemperatur unter Rühren 0,7 g (0,022 mol) 80 %iges Natriumhydrid und rührt die Mischung 16 Stunden bei Raumtemperatur nach. Zur Aufarbeitung gibt man auf halbkonzentrierte Ammoniumchlorid-Lösung und extrahiert zweimal mit jeweils 100 ml Methyl-t-butylether. Die vereinigten organischen Phasen werden getrocknet, im Vakuum eingeengt und der Rückstand durch Säulenchromatographie an Kieselgel mit (Dichlormethan/Essigsäureethylester 10:1) gereinigt.

Man erhält 6,9 g (74,5 % der Theorie) an E-3-Methoxy-2-{2-[6-(3-(5,6-dihydro-1,4,2-dioxazinyl)-phenoxy)-4-pyrimidinyloxy]-phenyl}-acrylsäuremethylester als Öl.
¹H-NMR (CDCl₃/TMS) δ (ppm): 3,61 (s, 3H), 3,75 (s, 3H), 4,20 (m, 2H), 4,51 (m, 2H),

### Beispiel 4

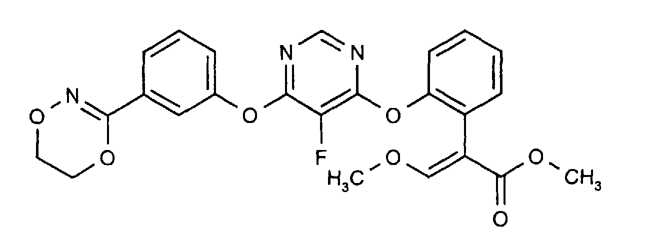

E-3-Methoxy-2-[2-(6-chlor-4-pyrimidinyloxy)-phenyl]-acrylsäuremethylester 6,4 g (0,02 mol) E-3-Methoxy-2-[2-(5-fluor-6-chlor-4-pyrimidinyloxy)-phenyl]-acrylsäure-methylester, 3,6 g (0,02 mol) 3-(3-Hydroxyphenyl)-5,6-dihydro-1,4,2-dioxazin und 2,7 g (0,024 mol) Kalium-tert-butanolat werden in 50 ml Tetrahydrofuran 4 Stunden lang unter Rückfluß erhitzt. Danach engt man die Lösung bis zur Trockne ein, nimmt in 50 ml Wasser auf und extrahiert mit Essigsäureethylester. Die organische Phase wird getrocknet und unter vermindertem Druck eingeengt. Zum Schluß reinigt man den Rückstand durch Säulenchromatographie an Kieselgel mit Dichlormethan/Essigsäureethylester (10:1). Man erhält 1,5 g (15,6 %) der Zielverbindung als hellgelbes Öl.
¹H-NMR (CDCl₃/TMS) δ (ppm): 3,63 (s,3H), 3,79 (s,3H), 4,11 (m,2H), 4,39 (m,2H),

Analog den Beispielen 1 bis 4, sowie entsprechend der allgemeinen Beschreibung der erfindungsgemäßen Herstellungsverfahren, können beispielsweise auch die in der nachstehenden Tabelle 2 aufgeführten Verbindungen hergestellt werden:

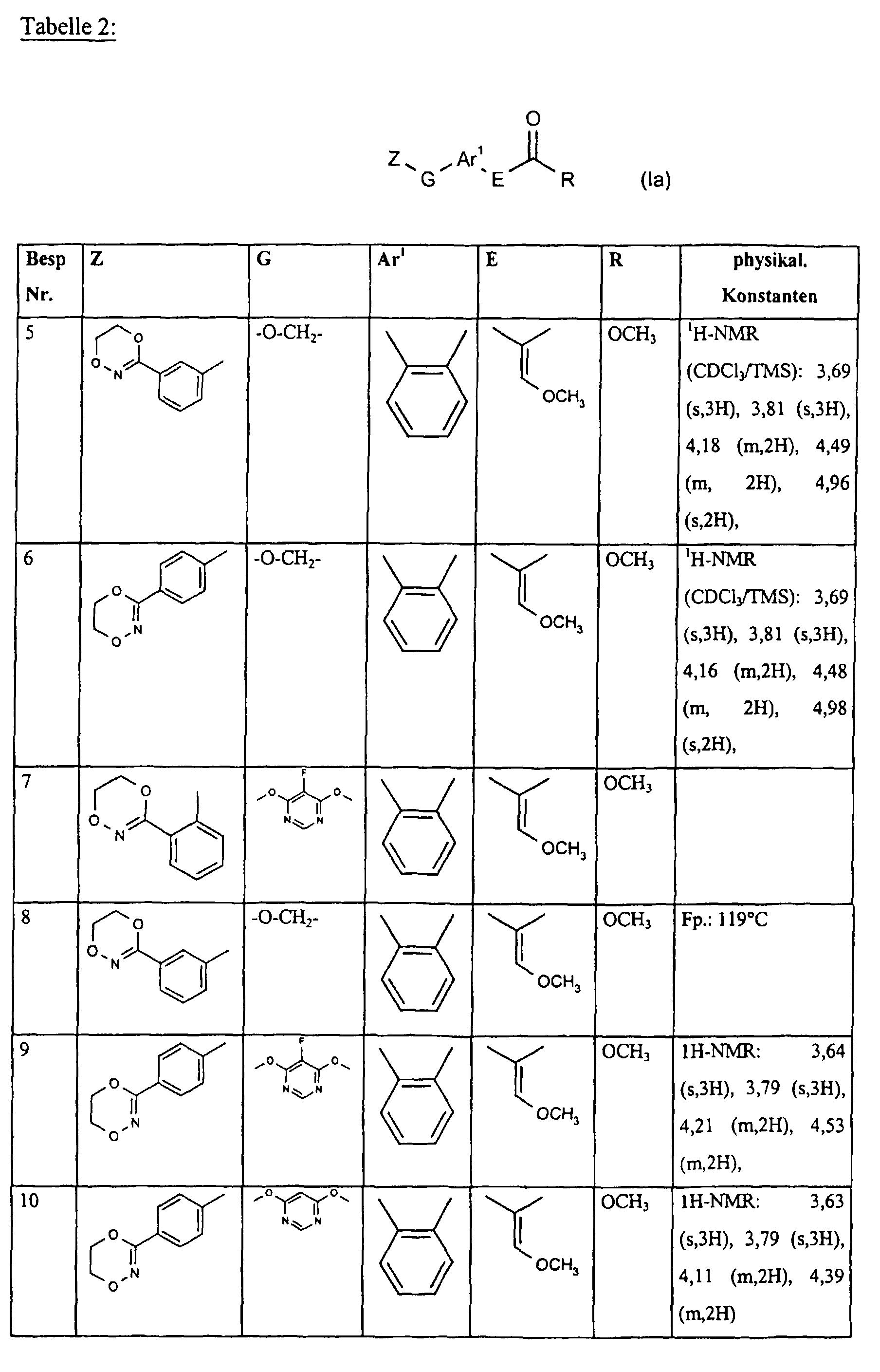

### Beispiel A

### Plasmopara-Test (Reben) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Feuchtkammer gestellt.

6 Tage nach der Inokulation erfolgt die Auswertung.

Bei diesem Test zeigen z.B. die folgenden Verbindungen (3), (5) und (8) bei einer Wirkstoffkonzentration von 100ppm einen Wirkungsgrad von bis zu 98 %.

### Beispiel B

### Podosphaera-Test (Apfel) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers Podosphaera leucotricha inokuliert.

Die Pflanzen werden dann im Gewächshaus bei 23°C und einer relativen Luftfeuchtigkeit von ca. 70 % aufgestellt.

10 Tage nach der Inokulation erfolgt die Auswertung.

Bei diesem Test zeigen z.B, die folgenden Verbindungen der Herstellungsbeispiele (2), (3), (5) und (7) bei einer Wirkstoffkonzentration von 100 ppm einen Wirkungsgrad von bis zu 100 %.

## Patentansprüche

1. Verbindungen der Formel (I) 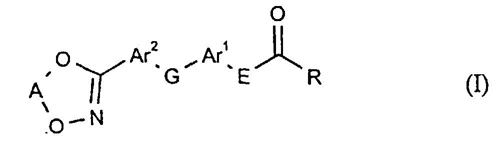 in welcher
A für -C₂H₄- steht,
Ar¹ für jeweils gegebenenfalls substituiertes Phenylen oder Naphthylen, für mono- oder bicyclisches Heteroarylen mit jeweils 5 oder 6 Ringgliedern oder für benzokondensiertes Heteroarylen mit 5 oder 6 Ringgliedern, von denen jeweils mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlebstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
Ar² für jeweils gegebenenfalls einfach oder mehrfach, gleich oder verschieden substituiertes Phenylen, Naphthylen oder für Heteroarylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
E für eine der nachstehenden Gruppierungen steht: 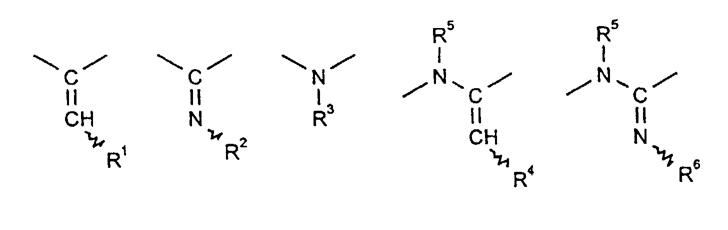 worin
R¹ für Wasserstoff, Halogen, Cyano oder für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylresten steht,
R² für Wasserstoff, Amino, Cyano oder für jeweils gegebenenfalls durch Halogen, Cyano oder C₁-C₄-Alkoxy substituiertes Alkyl, Alkoxy, Alkylamino oder Dialkylamino mit jeweils 1 bis 6 Kohlenstoffatomen in den Alkylresten steht,
R³ für Wasserstoff, Cyano oder für jeweils gegebenenfalls durch Halogen, Cyano oder C₁-C₄-Alkoxy substituiertes Alkyl, Alkenyl oder Alkinyl mit jeweils bis zu 6 Kohlenstoffatomen oder für jeweils gegebenenfalls durch Halogen, Cyano, Carboxy, C₁-C₄-Alkyl oder C₁-C₄-Alkoxycarbonyl substituiertes Cycloalkyl oder Cycloalkylalkyl mit 3 bis 6 Kohlenstoffatomen in den Cycloalkylteilen und gegebenenfalls 1 bis 4 Kohlenstoffatomen im Alkylteil steht,
R⁴ für Wasserstoff, Halogen, Cyano oder für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylresten steht,
R⁵ für Alkyl mit 1 bis 6 Kohlenstoffatomen steht und
R⁶ für Wasserstoff, Amino, Cyano oder für jeweils gegebenenfalls durch Halogen, Cyano oder C₁-C₄-Alkoxy substituiertes Alkyl, Alkoxy, Alkylamino oder Dialkylamino mit jeweils 1 bis 6 Kohlenstoffatomen in den Alkylresten steht;
G für eine Einfachbindung, für Sauerstoff, Schwefel oder für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁷)=N-O-, -C(R⁷)=N-O-CH₂-, -N(R⁸)-, -CQ-N(R⁸)-, -N(R⁸)-CQ-, -Q-CQ-N(R⁸)-, -N=C(R⁷)-Q-CH₂-, -CH₂-O-N=C(R⁷)-, -N(R⁸)-CQ-Q-, -CQ-N(R⁸)-CQ-Q-, -N(R⁸)-CQ-Q-CH₂-, -Q-C(R⁷)=N-O-CH₂-, -N(R⁸)-C(R⁷)=N-O-CH₂-, -O-CH₂-C(R⁷)=N-O-, -N=N-C(R⁷)-N-O-, -T-Ar³- oder -T-Ar³-Q- steht, wobei
n für die Zahlen 0, 1 oder 2 steht,
Q für Sauerstoff oder Schwefel steht,
R⁷ für Wasserstoff, Cyano, für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
R⁸ für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C1-C4-Alkoxycarbonyl substituiertes Cycloalkyl mit 3 bis 6 Kohlenstoffatomen steht und
Ar³ für jeweils gegebenenfalls einfach oder mehrfach, gleich oder verschieden substituiertes Phenylen, Naphthylen, Cycloalkylen oder für Heteroarylen oder Heterocycloalkylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, sowie;
Cycloalkyl mit 3 bis 6 Kohlenstoffatomen und
T für eine Einfachbindung, für Sauerstoff, Schwefel, -CH₂-O-, CH₂-S- oder für Alkandiyl mit 1 bis 3 Kohlenstoffatomen steht,
R für Alkyl, Alkoxy, Alkylamino, Hydroxylamino, Alkoxyamino oder Dialkylamino mit jeweils 1 bis 4 Kohlenstoffatomen steht.

2. Verbindungen der Formel (I) gemäß Anspruch 1, in welcher
Ar¹ für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Cyclopropyl, Trifluormethyl, Methoxy, Ethoxy, Methylthio, Methylsulfinyl oder Methylsulfonyl,
Ar² für jeweils gegebenenfalls einfach bis dreifach, gleich oder verschieden substituiertes Phenylen, Naphthylen, Furandiyl, Thiophendiyl, Oxazoldiyl, Isoxazoldiyl, Thiazoldiyl, Isothiazoldiyl, 1,2,4-Oxadiazoldiyl, 1,3,4-Oxadiazoldiyl, 1,2,4-Thiadiazoldiyl, 1,3,4-Thiadiazoldiyl, Pyridindiyl, Pyrimidindiyl, Pyridazindiyl, Pyrazindiyl, 1,2,3-Triazindiyl, 1,2,4-Triazindiyl, 1,3,5-Triazindiyl, steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s-oder t-Butyl, Cyclopropyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
E für eine der nachstehenden Gruppierungen steht: 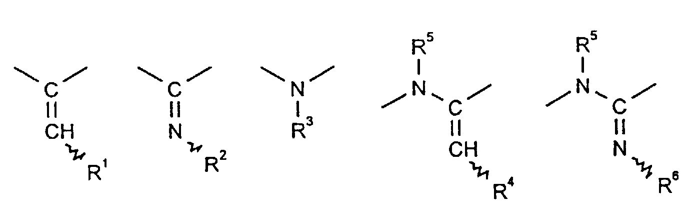 worin
R¹ für Wasserstoff, Fluor, Chlor, Brom, Cyano oder für jeweils gegebenenfalls durch Fluor, Chlor, Cyano, Methoxy oder Ethoxy substituiertes Methyl, Ethyl, Propyl, Methoxy, Ethoxy, Methylthio, Ethylthio, Methylamino, Ethylamino oder Dimethylamino steht,
R² für Wasserstoff, Amino, Cyano oder für jeweils gegebenenfalls durch Fluor, Chlor, Cyano, Methoxy oder Ethoxy substituiertes Methyl, Ethyl, Methoxy, Ethoxy, Methylamino, Ethylamino oder Dimethylamino steht,
R³ für Wasserstoff, Cyano oder für jeweils gegebenenfalls durch Fluor, Cyano, Methoxy oder Ethoxy substituiertes Methyl, Ethyl n- oder i-Propyl, n-, i- oder s-Butyl, für Allyl oder Propargyl oder für jeweils gegebenenfalls durch Fluor, Chlor, Cyano, Carboxy, Methyl, Ethyl, n-oder i-Propyl, Methoxy-carbonyl oder Ethoxy-carbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R⁴ für Wasserstoff, Fluor, Chlor, Brom, Cyano oder für jeweils gegebenenfalls durch Fluor, Chlor, Cyano, Methoxy oder Ethoxy substituiertes Methyl, Ethyl, Propyl, Methoxy, Ethoxy, Methylthio, Ethylthio, Methylamino, Ethylamino oder Dimethylamino steht,
R⁵ für Methyl, Ethyl n- oder i-Propyl, n-, i- oder s-Butyl steht,
R⁶ für Wasserstoff, Amino, Cyano oder für jeweils gegebenenfalls durch Fluor, Chlor, Cyano, Methoxy oder Ethoxy substituiertes Methyl, Ethyl, Methoxy, Ethoxy, Methylamino, Ethylamino oder Dimethylamino steht, und
G für eine Einfachbindung, für Sauerstoff, Schwefel oder für jeweils gegebenenfalls durch Fluor, Chlor, Brom, Hydroxy, Methyl, Ethyl, n-oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁷)=N-O-, -C(R⁷)=N-O-CH₂-, -N(R⁸)-, -CQ-N(R⁸)-, -N(R⁸)-CQ-, -Q-CQ-N(R⁸)-, -N=C(R⁷)-Q-CH₂-, -CH₂-O-N=C(R⁷)-, -N(R⁸)-CQ-Q-, -CQ-N(R⁸)-CQ-Q-, -N(R⁸)-CQ-Q-CH₂-, -Q-C(R⁷)=N-O-CH₂-, -N(R⁸)-C(R⁷)=N-O-CH₂-, -O-CH₂-C(R⁷)=N-O-, -N=N-C(R⁷)=N-O-, -T-Ar³- oder -T-Ar³-Q- steht, wobei
n für die Zahlen 0, 1 oder 2 steht,
Q für Sauerstoff oder Schwefel steht,
R⁷ für Wasserstoff, Cyano, für jeweils gegebenenfalls durch Fluor, Chlor, Cyano, Methoxy oder Ethoxy substituiertes Methyl, Ethyl, n- oder i-Propyl, n-, i- oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und
R⁸ für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für jeweils gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht,
Ar³ für jeweils gegebenenfalls einfach bis dreifach substituiertes Phenylen, Naphthylen, Furandiyl, Thiophendiyl, Oxazoldiyl, Isoxazoldiyl, Thiazoldiyl, Isothiazoldiyl, 1,2,4-Oxadiazoldiyl, 1,3,4-Oxadiazoldiyl, 1,2,4-Thiadiazoldiyl, 1,3,4-Thiadiazoldiyl, Pyridindiyl, Pyrimidindiyl, Pyridazindiyl, Pyrazindiyl, 1,2,3-Triazindiyl, 1,2,4-Triazindiyl, 1,3,5-Triazindiyl, Oxirandiyl, Oxetandiyl, Tetrahydrofurandiyl, Perhydropyrandiyl oder Pyrrolidindiyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl oder Cyclopropyl und
T für eine Einfachbindung, für Sauerstoff, Schwefel, -CH₂-O-, CH₂-S-, Methylen, Ethylen oder Propylen steht und
R für Methyl, Ethyl, n- oder i-Propyl, Methoxy, Ethoxy, n- oder i-Propoxy, Methylamino, Ethylarnino, n- oder i-Propylamino, Hydroxylamino, Methoxyamino, Dimethylamino, Diethylamino steht.

3. Verbindungen der allgemeinen Formel (I) gemäß Anspruch 1,
in welcher
Ar¹ für ortho-Phenylen, Pyridin-2,3-diyl oder Thiophen-2,3-diyl steht,
Ar² für jeweils gegebenenfalls einfach bis dreifach, gleich oder verschieden substituiertes Phenylen steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s-oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluomethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy,
E für eine der nachstehenden Gruppierungen steht 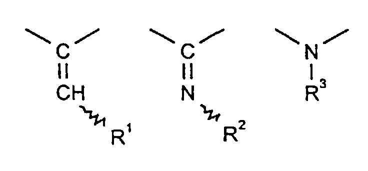 worin
R¹ und R² jeweils für Methoxy stehen und
R³ für Wasserstoff, Cyano, Methyl, Ethyl n- oder i-Propyl, Methoxy oder Ethoxy steht,
G für Sauerstoff oder für jeweils gegebenenfalls durch Fluor, Chlor oder Brom substituiertes Dimethylen (Ethan-1,2-diyl), Ethen-1,2-diyl oder eine der nachstehenden Gruppierungen
-Q-CQ-, -CQ-Q-, -CH₂-Q-; -Q-CH₂-, -CQ-Q-CH₂-,
-CH₂-Q-CQ-,
-Q-CQ-CH₂-, -Q-CQ-Q-CH₂-, -N=N-, -S(O)ₙ-, -CH₂-S(O)ₙ-,
-CQ-,
-S(O)ₙ-CH₂-, -C(R⁷)=N-O-, -C(R⁷)=N-O-CH₂-, -N(R⁸)-,
-CQ-N(R⁸)-,
-N(R⁸)-CQ-, -Q-CQ-N(R⁸)-, -N=C(R⁷)-Q-CH₂-,
-CH₂-O-N=C(R⁷)-,
-N(R⁸)-CQ-Q-, -CQ-N(R⁸)-CQ-Q-, -N(R⁸)-CQ-Q-CH₂-,
-Q-C(R⁷)=N-O-CH₂-, -N(R⁸)-C(R⁷)=N-O-CH₂-,
-O-CH₂-C(R⁷)=N-O-,
-N=N-C(R⁷)=N-O-, -T-Ar³- oder -T-Ar³-Q- steht, wobei
n für die Zahlen 0, 1 oder 2 steht,
Q für Sauerstoff oder Schwefel steht,
R⁷ für Wasserstoff, Cyano, Methyl, Ethyl oder Cyclopropyl steht und
R⁸ für Wasserstoff, Methyl, Ethyl oder Cyclopropyl steht,
Ar³ für jeweils gegebenenfalls einfach bis dreifach, gleich oder verschieden substituiertes Phenylen, 1,2,4-Thiadiazoldiyl, 1,3,4-Thiadiazoldiyl, 1,2,4-Oxadiazoldiyl, 1,3,4-Oxadiazoldiyl, Pyridindiyl, Pyrimidindiyl, Pyridazindiyl, Pyrazindiyl, 1,2,3-Triazindiyl, 1,2,4-Triazindiyl oder 1,3,5-Triazindiyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, Cyclopropyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl und
T für eine Einfachbindung, für Sauerstoff, Schwefel, -CH₂-O-, CH₂-S-, Methylen, Ethylen oder Propylen steht und
R für Methyl, Ethyl, Methoxy, Methylamino oder Hydroxylamino steht.

4. Verbindungen der Formel (I) gemäß Anspruch 1,
in welcher
Ar¹ für ortho-Phenylen, Pyridin-2,3-diyl oder Thiophen-2,3-diyl steht,
Ar² für jeweils gegebenenfalls einfach bis dreifach, gleich oder verschieden substituiertes Phenylen steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s-oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluomethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy,
E für eine der nachstehenden Gruppierungen steht, 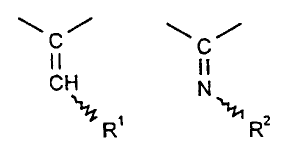 worin
R¹ und R² jeweils für Methoxy stehen und
G für -O-CH₂ steht und
R für Methyl, Ethyl, Methoxy, Methylamino oder Hydroxylamino steht.

5. Verbindungen der allgemeinen Formel (I)gemäß Anspruch 1,
in welcher
Ar¹ für ortho-Phenylen, Pyridin-2,3-diyl oder Thiophen-2,3-diyl steht,
Ar² für jeweils gegebenenfalls einfach bis dreifach, gleich oder verschieden substituiertes Phenylen steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s-oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluomethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy,
E für eine der nachstehenden Gruppierungen steht 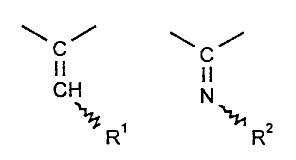 worin
R¹ und R² jeweils für Methoxy stehen und
G für -C(R⁷)=N-O-CH₂- steht, wobei
R⁷ für Wasserstoff, Cyano, Methyl, Ethyl oder Cyclopropyl steht und
R für Methyl, Ethyl, Methoxy, Methylamino oder Hydroxylamino steht.

6. Verbindungen der allgemeinen Formel (I) gemäß Anspruch 1,
in welcher
Ar¹ für ortho-Phenylen steht,
Ar² für jeweils gegebenenfalls einfach bis dreifach, gleich oder verschieden substituiertes Phenylen steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s-oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluomethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy,
E für eine der nachstehenden Gruppierungen steht: 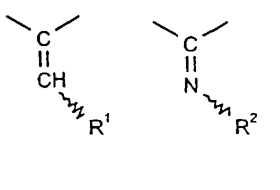 worin
R¹ und R² jeweils für Methoxy stehen,
G für -T-Ar³-Q- steht, wobei
Q für Sauerstoff oder Schwefel steht,
Ar³ für 1,2,4-Thiadiazoldiyl, 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio, Trifluormethyl, Difluormethoxy, Trifluormethoxy, Difluorchlormethoxy substituiertes, Pyridindiyl, Pyrimidindiyl oder 1,3,5-Triazindiyl steht,
T für eine Einfachbindung, für Sauerstoff, Schwefel, -CH₂-O-, CH₂-S-, Methylen, Ethylen oder Propylen steht und
R für Methyl, Ethyl, Methoxy, Methylarnino oder Hydroxylamino steht.

7. Schädlingsbekämpfungsmittel, gekennzeichnet durch einen Gehalt an einer Verbindung der Formel (I) nach Anspruch 1.

8. Verfahren zur Bekämpfung von Schädlingen, dadurch gekennzeichnet, daß man Verbindungen der Formel (I) nach Anspruch 1 auf Schädlinge und/oder ihren Lebensraum einwirken läßt.

9. Verwendung von Verbindungen der Formel (I) nach den Ansprüchen 1 bis 6 zur Bekämpfung von Schädlingen.

10. Verfahren zur Herstellung von Schädlingsbekämpfungsmitteln, dadurch gekennzeichnet, daß man Verbindungen der Formel (I) nach den Ansprüchen 1 bis 6 mit Streckmitteln und/oder oberflächenaktiven Mitteln vermischt.

11. Verfahren zur Herstellung von Verbindungen der Formel (I) nach Anspruch 1,
dadurch gekennzeichnet, daß man
a) Hydroxyverbindungen der allgemeinen Formel (II) 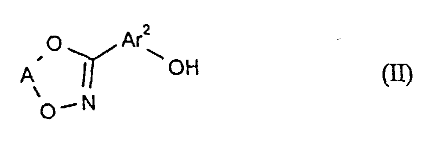 in welcher
A und Ar² die in Anspruch 1 angegebenen Bedeutungen haben,
mit Halogenverbindungen der allgemeinen Formel (III) 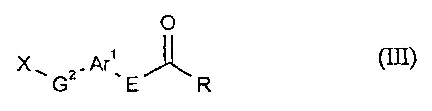 in welcher
G² für -Ar³-Q- oder für -Ar³- steht,
Ar¹, Ar³, E, Q und R die in Anspruch 1 angegebenen Bedeutungen haben und
X für Halogen steht,
gegebenenfalls in Gegenwart eines Säureakzeptors und gegebenenfalls in Gegenwart eines Verdünnungsmittels umsetzt;
b) Hydroxyverbindungen der allgemeinen Formel (II) 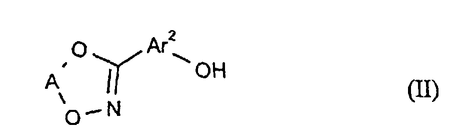 in welcher
A und Ar² die in Anspruch 1 angegebenen Bedeutungen haben,
mit Halogenverbindungen der allgemeinen Formel (IV) 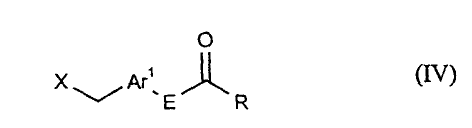 in welcher
Ar¹, E, und R die in Anspruch 1 angegebenen Bedeutungen haben und
X für Halogen steht,
gegebenenfalls in Gegenwart eines Säureakzeptors und gegebenenfalls in Gegenwart eines Verdünnungsmittels umsetzt;
c) Ester der allgemeinen Formel (I-1) 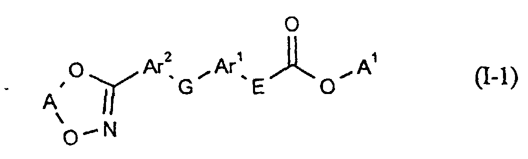 in welcher
A, Ar¹, Ar², E und G die in Anspruch 1 angegebenen Bedeutungen haben und
A¹ für Alkyl steht,
mit Aminen der allgemeinen Formel (V), 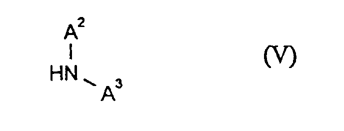 in welcher
A² und A³ unabhängig voneinander jeweils für Wasserstoff, jeweils gegebenenfalls substituiertes Alkyl, Alkoxy oder Hydroxy stehen,
- oder mit einem Säureadditionskomplex hiervon -
gegebenenfalls in Gegenwart eines Säureakzeptors und gegebenenfalls in Gegenwart eines Verdünnungsmittels umsetzt.

## Claims

1. Compounds of the formula (1) 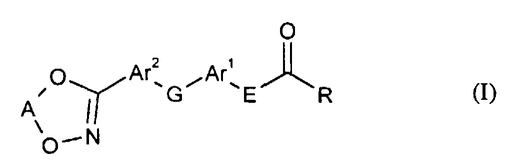 in which
A represents -C₂H₄-,
Ar¹ represents respectively optionally substituted phenylene or naphthylene, represents mono- or dicyclic heteroarylene having in each case 5 or 6 ring members or represents benzo-fused heteroarylene having 5 or 6 ring members, at least one of which in each case is oxygen, sulphur or nitrogen and one or two more of which optionally are nitrogen, the possible substituents preferably being selected from the list below:
halogen, cyano, nitro, amino, hydroxyl, formyl, carboxyl, carbamoyl, thiocarbamoyl, respectively straight-chain or branched alkyl, alkoxy, alkylthio, alkylsulphinyl or alkylsulphonyl having in each case 1 to 6 carbon atoms, respectively straight-chain or branched alkenyl, alkenyloxy or alkinyloxy having in each case 2 to 6 carbon atoms, respectively straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms, respectively straight-chain or branched halogenoalkenyl or halogenoalkenyloxy having in each case 2 to 6 carbon atoms and 1 to 13 identical or different halogen atoms, respectively straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties, alkylene or dioxyalkylene, each of which has 1 to 6 carbon atoms, is attached twice and is optionally mono- or polysubstituted by identical or different substituents from the group consisting of halogen, straight-chain or branched alkyl having 1 to 4 carbon atoms and straight-chain or branched halogenoalkyl having 1 to 4 carbon atoms and 1 to 9 identical or different halogen atoms,
Ar² represents phenylene or naphthylene or represents heteroarylene having 3 to 7 ring members, each of the radicals being optionally mono- or polysubstituted by identical or different substituents and at least one of the 3 to 7 ring members being oxygen, sulphur or nitrogen and one or two more optionally being nitrogen, the possible substituents preferably being selected from the list below:
halogen, cyano, nitro, amino, hydroxyl, formyl, carboxyl, carbamoyl, thiocarbamoyl;
respectively straight-chain or branched alkyl, alkoxy, alkylthio, alkylsulphinyl or alkylsulphonyl having in each case 1 to 6 carbon atoms;
respectively straight-chain or branched alkenyl or alkenyloxy having in each case 2 to 6 carbon atoms;
respectively straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
respectively straight-chain or branched halogenoalkenyl or halogenoalkenyloxy having in each case 2 to 6 carbon atoms and 1 to 13 identical or different halogen atoms;
respectively straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
alkylene or dioxyalkylene, each of which has 1 to 6 carbon atoms, is attached twice and is optionally mono- or polysubstituted by identical or different substituents from the group consisting of halogen, straight-chain or branched alkyl having 1 to 4 carbon atoms and straight-chain or branched halogenoalkyl having 1 to 4 carbon atoms and 1 to 9 identical or different halogen atoms;
cycloalkyl having 3 to 6 carbon atoms;
E represents one of the groupings below: 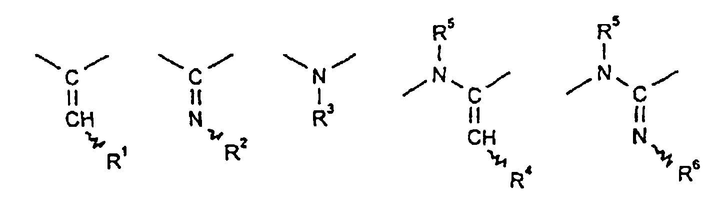 in which
R¹ represents hydrogen, halogen, cyano or represents respectively optionally halogen-, cyano- or C₁-C₄-alkoxy-substituted alkyl, alkoxy, alkylthio, alkylamino or dialkylamino having in each case 1 to 6 carbon atoms in the alkyl radicals,
R² represents hydrogen, amino, cyano or represents respectively optionally halogen-, cyano- or C₁-C₄-alkoxy-substituted alkyl, alkoxy, alkylamino or dialkylamino having in each case 1 to 6 carbon atoms in the alkyl radicals,
R³ represents hydrogen, cyano or represents respectively optionally halogen-, cyano- or C₁-C₄-alkoxy-substituted alkyl, alkenyl or alkinyl having in each case up to 6 carbon atoms or represents respectively optionally halogen-, cyano-, carboxyl-, C₁-C₄-alkyl- or C₁-C₄-alkoxy-carbonyl-substituted cycloalkyl or cycloalkylalkyl having 3 to 6 carbon atoms in the cycloalkyl moieties and optionally 1 to 4 carbon atoms in the alkyl moiety,
R⁴ represents hydrogen, halogen, cyano or represents respectively optionally halogen-, cyano- or C₁-C₄-alkoxy-substituted alkyl, alkoxy, alkylthio, alkylamino or dialkylamino having in each case 1 to 6 carbon atoms in the alkyl radicals,
R⁵ represents alkyl having 1 to 6 carbon atoms and
R⁶ represents hydrogen, amino, cyano or represents respectively optionally halogen-, cyano- or C₁-C₄-alkoxy-substituted alkyl, alkoxy, alkylamino or dialkylamino having in each case 1 to 6 carbon atoms in the alkyl radicals;
G represents a single bond, represents oxygen, sulphur or represents respectively optionally halogen-, hydroxyl-, C₁-C₄-alkyl-, C₁-C₄-halogenoalkyl- or C₃-C₆-cycloalkyl-substituted alkanediyl, alkenediyl, alkinediyl having in each case up to 4 carbon atoms or one of the groupings below
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁷)=N-O-, -C(R⁷)=N-O-CH₂-, -N(R⁸)-, -CQ-N(R⁸)-, -N(R⁸)-CQ-, -Q-CQ-N(R⁸)-, -N=C(R⁷)-Q-CH₂-, -CH₂-O-N=C(R⁷)-, -N(R⁸)-CQ-Q-, -CQ-N(R⁸)-CQ-Q-, -N(R⁸)-CQ-Q-CH₂-, -Q-C(R⁷)=N-O-CH₂-, -N(R⁸)-C(R⁷)=N-O-CH₂-, -O-CH₂-C(R⁷)=N-O-, -N=N-C(R⁷)=N-O-, -T-Ar³-or -T-Ar³-Q-, where
n represents the numbers 0, 1 or 2,
Q represents oxygen or sulphur,
R⁷ represents hydrogen, cyano, represents respectively optionally halogen-, cyano- or C₁-C₄-alkoxy-substituted alkyl, alkoxy, alkylthio, alkylamino or dialkylamino having in each case 1 to 6 carbon atoms in the alkyl groups, or represents respectively optionally halogen-, cyano-, carboxyl-, C₁-C₄-alkyl or C₁-C₄-alkoxy-carbonyl-substituted cycloalkyl having 3 to 6 carbon atoms, and
R⁸ represents hydrogen, hydroxyl, cyano or represents optionally halogen-, cyano- or C₁-C₄-alkoxy-substituted alkyl having 1 to 6 carbon atoms, or represents optionally halogen-, cyano-, carboxyl-, C₁-C₄-alkyl- or C₁-C₄-alkoxy-carbonyl-substituted cycloalkyl having 3 to 6 carbon atoms and
Ar³ represents phenylene, naphthylene, cycloalkylene or represents heteroarylene or heterocycloalkylene having 3 to 7 ring members, each of the radicals being optionally mono- or polysubstituted by identical or different substituents and at least one of the 3 to 7 ring members being oxygen, sulphur or nitrogen and one or two more optionally being nitrogen, the possible substituents preferably being selected from the list below:
halogen, cyano, nitro, amino, hydroxyl, formyl, carboxyl, carbamoyl, thiocarbamoyl;
respectively straight-chain or branched alkyl, alkoxy, alkylthio, alkylsulphinyl or alkylsulphonyl having in each case 1 to 6 carbon atoms;
respectively straight-chain or branched alkenyl or alkenyloxy having in each case 2 to 6 carbon atoms;
respectively straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
respectively straight-chain or branched halogenoalkenyl or halogenoalkenyloxy having in each case 2 to 6 carbon atoms and I to 13 identical or different halogen atoms;
respectively straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties, and;
cycloalkyl having 3 to 6 carbon atoms and
T represents a single bond, represents oxygen, sulphur -CH₂-O-, CH₂-S- or represents alkanediyl having 1 to 3 carbon atoms,
R represents alkyl, alkoxy, alkylamino, hydroxylamino, alkoxyamino or dialkylamino having in each case 1 to 4 carbon atoms.

2. Compounds of the formula (I) according to Claim 1 in which
Ar¹ represents respectively optionally substituted ortho-, meta- orpara-phenylene, represents furanediyl, thiophenediyl, pyrrolediyl, pyrazolediyl, triazolediyl, oxazolediyl, isoxazolediyl, thiazolediyl, isothiazolediyl, oxadiazolediyl, thiadiazolediyl, pyridinediyl (in particular pyridine-2,3-diyl), pyrimidinediyl, pyridazinediyl, pyrazinediyl, 1,3,4-triazinediyl or 1,2,3-triazinediyl, the possible substituents in particular being selected from the list below:
fluorine, chlorine, cyano, methyl, ethyl, cyclopropyl, trifluoromethyl, methoxy, ethoxy, methylthio, methylsulphinyl or methylsulphonyl,
Ar² represents phenylene, naphthylene, furanediyl, thiophenediyl, oxazolediyl, isoxazolediyl, thiazolediyl, isothiazolediyl, 1,2,4-oxadiazolediyl, 1,3,4-oxadiazolediyl, 1,2,4-thiadiazolediyl, 1,3,4-thiadiazolediyl, pyridinediyl, pyrimidinediyl, pyridazinediyl, pyrazinediyl, 1,2,3-triazinediyl, 1,2,4-triazinediyl, 1,3,5-triazinediyl, each of which is optionally mono- to trisubstituted by identical or different substituents, the possible substituents preferably being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, cyclopropyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl;
E represents one of the groupings below: 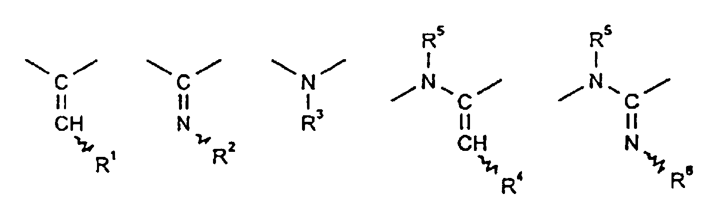 in which
R¹ represents hydrogen, fluorine, chlorine, bromine, cyano or represents respectively optionally fluorine-, chlorine-, cyano-, methoxy- or ethoxy-substituted methyl, ethyl, propyl, methoxy, ethoxy, methylthio, ethylthio, methylamino, ethylamino or dimethylamino,
R² represents hydrogen, amino, cyano or represents respectively optionally fluorine-, chlorine-, cyano-, methoxy- or ethoxy-substituted methyl, ethyl, methoxy, ethoxy, methylamino, ethylamino or dimethylamino,
R³ represents hydrogen, cyano or represents respectively optionally fluorine-, cyano-, methoxy- or ethoxy-substituted methyl, ethyl, n- or i-propyl, n-, i- or s-butyl, represents allyl or propargyl or represents respectively optionally fluorine-, chlorine-, cyano-, carboxyl-, methyl-, ethyl-, n- or i-propyl-, methoxy-carbonyl- or ethoxy-carbonyl-substituted cyclopropyl, cyclobutyl, cyclopentyl, cyclohexyl, cyclopropylmethyl, cyclobutylmethyl, cyclopentylmethyl or cyclohexylmethyl,
R⁴ represents hydrogen, fluorine, chlorine, bromine, cyano or represents respectively optionally fluorine-, chlorine-, cyano-, methoxy- or ethoxy-substituted methyl, ethyl, propyl, methoxy, ethoxy, methylthio, ethylthio, methylamino, ethylamino or dimethylamino,
R⁵ represents methyl, ethyl, n- or i-propyl, n-, i- or s-butyl,
R⁶ represents hydrogen, amino, cyano or represents respectively optionally fluorine-, chlorine-, cyano-, methoxy- or ethoxy-substituted methyl, ethyl, methoxy, ethoxy, methylamino, ethylamino or dimethylamino, and
G represents a single bond, represents oxygen, sulphur, or represents respectively optionally fluorine-, chlorine-, bromine-, hydroxyl-, methyl-, ethyl-, n- or i-propyl-, trifluoromethyl-, cyclopropyl-, cyclobutyl-, cyclopentyl- or cyclohexyl-substituted methylene, dimethylene (ethane-1,2-diyl), ethene-1,2-diyl, ethine-1,2-diyl or one of the groupings below
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁷)=N-O-, -C(R⁷)=N-O-CH₂-, -N(R⁸)-, -CQ-N(R⁸)-, -N(R⁸)-CQ-, -Q-CQ-N(R⁸)-, -N=C(R⁷)-Q-CH₂-, -CH₂-O-N=C(R⁷)-, -N(R⁸)-CQ-Q-, -CQ-N(R⁸)-CQ-Q-, -N(R⁸)-CQ-Q-CH₂-, -Q-C(R⁷)=N-O-CH₂-, -N(R⁸)-C(R⁷)=N-O-CH₂-, -O-CH₂-C(R⁷)=N-O-, -N=N-C(R⁷)=N-O-, -T-Ar³-or -T-Ar³-Q-, where
n represents the numbers 0, 1 or 2,
Q represents oxygen or sulphur,
R⁷ represents hydrogen, cyano, represents respectively optionally fluorine-, chlorine-, cyano-, methoxy- or ethoxy-substituted methyl, ethyl, n- or i-propyl, n-, i- or s-butyl, methoxy, ethoxy, propoxy, butoxy, methylthio, ethylthio, propylthio, butylthio, methylamino, ethylamino, propylamino, dimethylamino or diethylamino or represents respectively optionally fluorine-, chlorine-, cyano-, carboxyl-, methyl-, ethyl-, n- or i-propyl-, methoxycarbonyl- or ethoxy-carbonyl-substituted cyclopropyl, cyclobutyl, cyclopentyl or cyclohexyl, and
R⁸ represents hydrogen, hydroxyl, cyano or represents respectively optionally fluorine-, chlorine-, cyano-, methoxy- or ethoxy-substituted methyl, ethyl, n- or i-propyl, n-, i-, s- or t-butyl or represents respectively optionally fluorine-, chlorine-, cyano-, carboxyl-, methyl-, ethyl-, n- or i-propyl-, methoxy-carbonyl- or ethoxy-carbonyl-substituted cyclopropyl, cyclobutyl, cyclopentyl or cyclohexyl,
Ar³ represents phenylene, naphthylene, furanediyl, thiophenediyl, oxazolediyl, isoxazolediyl, thiazolediyl, isothiazolediyl, 1,2,4-oxadiazolediyl, 1,3,4-oxadiazolediyl, 1,2,4-thiadiazolediyl, 1,3,4-thiadiazolediyl, pyridinediyl, pyrimidinediyl, pyridazinediyl, pyrazinediyl, 1,2,3-triazinediyl, 1,2,4-triazinediyl, 1,3,5-triazinediyl, oxiranediyl, oxetanediyl, tetrahydrofuranediyl, perhydropyranediyl or pyrrolidinediyl, each of which is optionally mono- to trisubstituted, the possible substituents preferably being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, , acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl, ethoximinoethyl or cyclopropyl and
T represents a single bond, represents oxygen, sulphur, -CH₂-O-, CH₂-S-, methylene, ethylene or propylene and
R represents methyl, ethyl, n- or i-propyl, methoxy, ethoxy, n- or i-propoxy, methylamino, ethylamino, n- or i-propylamino, hydroxylamino, methoxyamino, dimethylamino, diethylamino.

3. Compounds of the general formula (I) according to Claim 1
in which
Ar¹ represents ortho-phenylene, pyridine-2,3-diyl or thiophene-2,3-diyl,
Ar² represents phenylene which is respectively optionally mono- to trisubstituted by identical or different substituents, the possible substituents preferably being selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl, methoximinomethyl, ethoximinomethyl, methoximinoethyl, ethoximinoethyl,
methylenedioxy or ethylenedioxy, each of which is attached twice and each of which is optionally mono- to tetrasubstituted by identical or different substituents from the group consisting of fluorine, chlorine, methyl, trifluoromethyl or ethyl,
E represents one of the groupings below 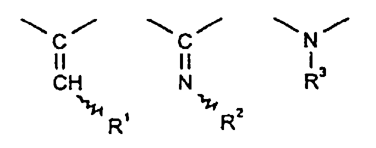 in which
R¹ and R² each represent methoxy and
R³ represents hydrogen, cyano, methyl, ethyl, n- or i-propyl, methoxy or ethoxy,
G represents oxygen or represents respectively optionally fluorine-, chlorine-or bromine-substituted dimethylene (ethane-1,2-diyl), ethene-1,2-diyl or one of the groupings below
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁷)=N-O-, -C(R⁷)=N-O-CH₂-, -N(R⁸)-, -CQ-N(R⁸)-, -N(R⁸)-CQ-, -Q-CQ-N(R⁸)-, -N=C(R⁷)-Q-CH₂-, -CH₂-O-N=C(R⁷)-, -N(R⁸)-CQ-Q-, -CQ-N(R⁸)-CQ-Q-, -N(R⁸)-CQ-Q-CH₂-, -Q-C(R⁷)=N-O-CH₂-, -N(R⁸)-C(R⁷)=N-O-CH₂-, -O-CH₂-C(R⁷)=N-O-, -N=N-C(R⁷)=N-O-, -T-Ar³- or -T-Ar³-Q-, where
n represents the numbers 0, 1 or 2,
Q represents oxygen or sulphur,
R⁷ represents hydrogen, cyano, methyl, ethyl or cyclopropyl and
R⁸ represents hydrogen, methyl, ethyl or cyclopropyl,
Ar³ represents phenylene, 1,2,4-thiadiazolediyl, 1,3,4-thiadiazolediyl, 1,2,4-oxadiazolediyl, 1,3,4-oxadiazolediyl, pyridinediyl, pyrimidinediyl, pyridazinediyl pyrazinediyl, 1,2,3-triazinediyl, 1,2,4-triazinediyl or 1,3,5-triazinediyl, each of which is optionally mono- to trisubstituted by identical or different substituents, the possible substituents preferably being selected from the list below:
fluorine, chlorine, bromine, cyano, methyl, ethyl, n- or i-propyl, cyclopropyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, and
T represents a single bond, represents oxygen, sulphur, -CH₂-O-, CH₂-S-, methylene, ethylene or propylene and
R represents methyl, ethyl, methoxy, methylamino or hydroxylamino,

4. Compounds of the formula (I) according to Claim 1
in which
Ar¹ represents ortho-phenylene, pyridine-2,3-diyl or thiophene-2,3-diyl,
Ar² represents phenylene which is in each case optionally mono- to trisubstituted by identical or different substituents, the possible substituents preferably being selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl, methoximinomethyl, ethoximinomethyl, methoximinoethyl, ethoximinoethyl,
methylenedioxy or ethylenedioxy, each of which is attached twice and each of which is optionally mono- to tetrasubstituted by identical or different substituents from the group consisting of fluorine, chlorine, methyl, trifluoromethyl or ethyl,
E represents one of the groupings below 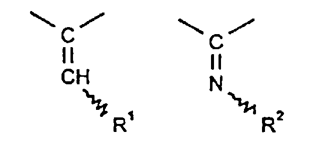 in which
R¹ and R² each represent methoxy and
G represents -O-CH₂ and
R represents methyl, ethyl, methoxy, methylamino or hydroxylamino.

5. Compounds of the general formula (I) according to Claim 1
in which
Ar¹ represents ortho-phenylene, pyridine-2,3-diyl or thiophene-2,3-diyl,
Ar² represents phenylene which is in each case optionally mono- to trisubstituted by identical or different substituents, the possible substituents preferably being selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl, methoximinomethyl, ethoximinomethyl, methoximinoethyl, ethoximinoethyl,
methylenedioxy or ethylenedioxy, each of which is attached twice and each of which is optionally mono- to tetrasubstituted by identical or different substituents from the group consisting of fluorine, chlorine, methyl, trifluoromethyl or ethyl,
E represents one of the groupings below 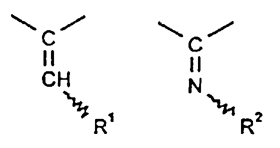 in which
R¹ and R² each represent methoxy and
G represents -C(R⁷)=N-O-CH₂- where
R⁷ represents hydrogen, cyano, methyl, ethyl or cyclopropyl and
R represents methyl, ethyl, methoxy, methylamino or hydroxylamino.

6. Compounds of the general formula (I) according to Claim 1
in which
Ar¹ represents ortho-phenylene,
Ar² represents phenylene which is in each case optionally mono- to trisubstituted by identical or different substituents, the possible substituents preferably being selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluomethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl, methoximinomethyl, ethoximinomethyl, methoximinoethyl, ethoximinoethyl,
methylenedioxy or ethylenedioxy, each of which is attached twice and each of which is optionally mono- to tetrasubstituted by identical or different substituents from the group consisting of fluorine, chlorine, methyl, trifluoromethyl or ethyl,
E represents one of the groupings below: 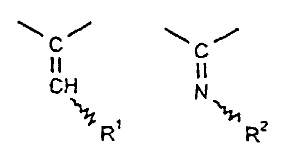 in which
R¹ and R² each represent methoxy,
G represents -T-Ar³-Q- where
Q represents oxygen or sulphur,
Ar³ represents 1,2,4-thiadiazolediyl, 1,3,4-thiadiazolediyl, 1,2,4-oxadiazolediyl, 1,3,4-oxadiazolediyl or represents pyridinediyl, pyrimidinediyl or 1,3,5-triazinediyl, each of which is optionally mono- or disubstituted by identical or different substituents from the group consisting of fluorine, chlorine, cyano, methyl, cyclopropyl, methoxy, methylthio, trifluoromethyl, difluoromethoxy, trifluoromethoxy, difluorochloromethoxy,
T represents a single bond, represents oxygen, sulphur, -CH₂-O-, CH₂-S-, methylene, ethylene or propylene and
R represents methyl, ethyl, methoxy, methylamino or hydroxylamino.

7. Pesticides, characterized in that they comprise a compound of the formula (I) according to Claim 1.

8. Method for controlling pests, characterized in that compounds of the formula (I) according to Claim 1 are allowed to act on pests and/or their habitat.

9. Use of compounds of the formula (I) according to Claims 1 to 6 for controlling pests.

10. Process for preparing pesticides, characterized in that compounds of the formula (I) according to Claims 1 to 6 are mixed with extenders and/or surface-active agents.

11. Process for preparing compounds of the formula (I) according to Claim 1, characterized in that
a) hydroxyl compounds of the general formula (II) 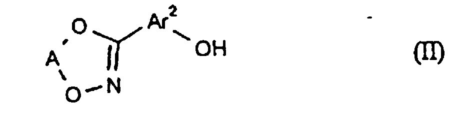 in which
A and Ar² are each as defined in Claim 1,
are reacted with halogen compounds of the general formula (III) 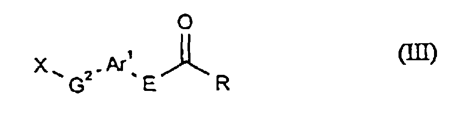 in which
G² represents -Ar³-Q- or represents -Ar³-,
Ar¹, Ar³, E, Q and R are each as defined in Claim 1 and
X represents halogen,
if appropriate in the presence of an acid acceptor and if appropriate in the presence of a diluent;
b) hydroxyl compounds of the general formula (II) 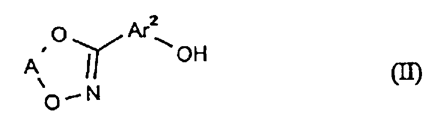 in which
A and Ar² are each as defined in Claim 1,
are reacted with halogen compounds of the general formula (IV) 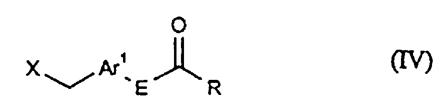 in which
Ar¹, E and R are each as defined in Claim 1 and
X represents halogen,
if appropriate in the presence of an acid acceptor and if appropriate in the presence of a diluent;
c) esters of the general formula (I-1) 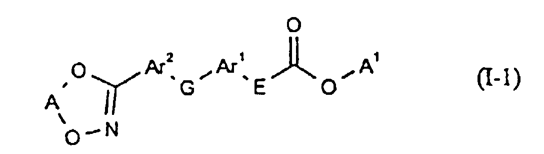 in which
A, Ar¹, Ar², E and G are each as defined in Claim 1 and
A¹ represents alkyl,
are reacted with amines of the general formula (V), 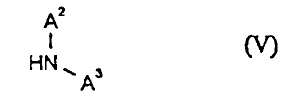 in which
A² and A³ independently of one another are each hydrogen, respectively optionally substituted alkyl, alkoxy or hydroxyl,
- or an acid addition complex thereof -
if appropriate in the presence of an acid acceptor and if appropriate in the presence of a diluent.

## Revendications

1. Composés de formule (I) 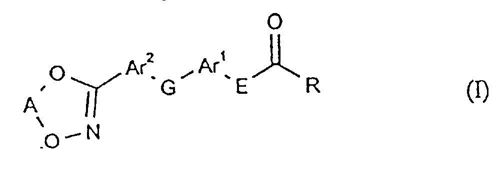 dans laquelle
A est un groupe -C₂H₄-,
Ar¹ est un groupe phénylène ou naphtylène chacun éventuellement substitué, un groupe hétéroarylène mono- ou bicyclique ayant dans chaque cas 5 ou 6 atomes ou un groupe hétéroarylène condensé au benzène de 5 ou 6 atomes, dont l'un au moins dans chaque cas est de l'oxygène, du soufre ou de l'azote et, le cas échéant, un ou deux autres représentent de l'azote, les substituants possibles étant choisis de préférence parmi ceux qui sont énumérés ci-après :
halogène, cyano, nitro, amino, hydroxy, formyle, carboxy, carbamoyle, thiocarbamoyle, alkyle, alkoxy, alkylthio, alkylsulfinyle ou alkylsulfonyle chacun linéaire ou ramifié et ayant chacun 1 à 6 atomes de carbone, alcényle, alcényloxy ou alcynyloxy chacun linéaire ou ramifié ayant chacun 2 à 6 atomes de carbone, halogénalkyle, halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents, halogénalcényle ou halogénalcényloxy chacun linéaire ou ramifié ayant chacun 2 à 6 atomes de carbone et 1 à 13 atomes d'halogènes identiques ou différents, alkylamino, dialkylamino, alkylcarbonyle, alkylcarbonyloxy, alkoxycarbonyle, alkylsulfonyloxy, hydroximinoalkyle ou alkoximinoalkyle, chacun linéaire ou ramifié ayant chacun 1 à 6 atomes de carbone dans les parties alkyle individuelles, alkylène ou dioxyalkylène ayant chacun deux liaisons et 1 à 6 atomes de carbone, chacun étant éventuellement substitué une ou plusieurs fois identiques ou différentes par un halogène et/ou par un radical alkyle linéaire ou ramifié ayant 1 à 4 atomes de carbone et/ou par un radical halogénalkyle linéaire ou ramifié ayant 1 à 4 atomes de carbone et 1 à 9 atomes d'halogènes identiques ou différents,
Ar² est un groupe phénylène ou naphtylène portant éventuellement chacun un ou plusieurs substituants identiques ou différents ou un groupe hétéroarylène de 3 à 7 atomes dont l'un au moins est un atome d'oxygène, de soufre ou d'azote et le cas échéant un ou deux autres représentent de l'azote, les substituants possibles étant choisis de préférence parmi ceux qui sont énumérés ci-après :
halogène, cyano, nitro, amino, hydroxy, formyle, carboxy, carbamoyle, thiocarbamoyle ;
alkyle, alkoxy, alkylthio, alkylsulfinyle ou alkylsulfonyle chacun linéaire ou ramifié ayant chacun 1 à 6 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié et ayant chacun 1 à 6 atomes de carbone et 1 à 13 atomes d'halogènes identiques ou différents ;
halogénalcényle ou halogénalcényloxy chacun linéaire ou ramifié et ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximinoalkyle ou alkoximinoalkyle chacun linéaire ou ramifié et ayant chacun 1 à 6 atomes de carbone dans les parties alkyle individuelles ;
alkylène ou dioxyalkylène ayant chacun deux liaisons et chacun 1 à 6 atomes de carbone, chacun étant éventuellement substitué une ou plusieurs fois identiques ou différentes par un halogène et/ou par un radical alkyle linéaire ou ramifié ayant 1 à 4 atomes de carbone et/ou par un radical halogénalkyle linéaire ou ramifié ayant 1 à 4 atomes de carbone et 1 à 9 atomes d'halogènes identiques ou différents ;
cycloalkyle ayant 3 à 6 atomes de carbone ;
E représente l'un des groupements suivants : 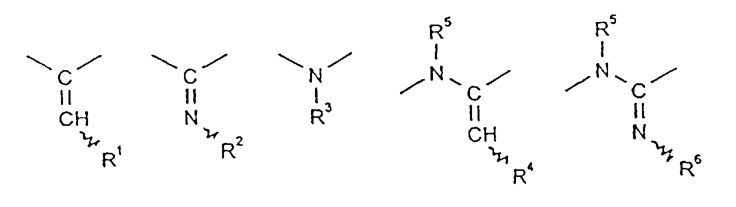 dans lesquels
R¹ représente l'hydrogène, un halogène, un groupe cyano ou un groupe alkyle, alkoxy, alkylthio, alkylamino ou dialkylamino ayant chacun 1 à 6 atomes de carbone dans les restes alkyle et chacun étant éventuellement substitué par un radical halogéno, cyano ou alkoxy en C₁ à C₄,
R² est l'hydrogène, un groupe amino, cyano, ou un groupe alkyle, alkoxy, alkylamino ou dialkylamino ayant chacun 1 à 6 atomes de carbone dans les restes alkyle et chacun étant éventuellement substitué par un radical halogéno, cyano ou alkoxy en C₁ à C₄,
R³ est l'hydrogène, un groupe cyano ou un groupe alkyle, alcényle ou alcynyle ayant chacun jusqu'à 6 atomes de carbone et chacun étant éventuellement substitué par un halogène, un radical cyano ou alkoxy en C₁ à C₄, ou un groupe cycloalkyle ou cycloalkylalkyle ayant 3 à 6 atomes de carbone dans les parties cycloalkyle et le cas échéant 1 à 4 atomes de carbone dans la partie alkyle et chacun étant éventuellement substitué par un radical halogéno, cyano, carboxy, alkyle en C₁ à C₄ ou (alkoxy en C₁ à C₄) -carbonyle,
R⁴ représente l'hydrogène, un halogène, un groupe cyano ou un groupe alkyle, alkoxy, alkylthio, alkylamino ou dialkylamino ayant chacun 1 à 6 atomes de carbone dans les restes alkyle et chacun étant éventuellement substitué par un radical halogéno, cyano ou alkoxy en C₁ à C₄,
R⁵ est un groupe alkyle ayant 1 à 6 atomes de carbone et
R⁶ représente l'hydrogène, un groupe amino ou cyano ou un groupe alkyle, alkoxy, alkylamino ou dialkylamino ayant chacun 1 à 6 atomes de carbone dans les restes alkyle et chacun étant éventuellement substitué par un radical halogéno, cyano ou alkoxy en C₁ à C₄ ;
G représente une liaison simple, l'oxygène, le soufre ou un groupe alcanediyle, alcènediyle, alcynediyle ayant dans chaque cas jusqu'à 4 atomes de carbone et chacun étant éventuellement substitué par un radical halogéno, hydroxy, alkyle en C₁ à C₄, halogénalkyle en C₁ à C₄ ou cycloalkyle en C₃ à C₆, ou l'un des groupements suivants
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁷)=N-O-, -C(R⁷)=N-O-CH₂-, -N(R⁸)-, -CQ-N(R⁸)-, -N(R⁸)-CQ-, -Q-CQ-N(R⁸)-, -N=C(R⁷)-Q-CH₂-, -CH₂-O-N=C(R⁷)-, -N(R⁸)-CQ-Q-, -CQ-N(R⁸)-CQ-Q-, -N(R⁸)-CQ-Q-CH₂-, -Q-C(R⁷)=N-O-CH₂-, -N(R⁸)-C(R⁷)=N-O-CH₂-, -O-CH₂-C(R⁷)=N-O-, -N=N-C(R⁷)=N-O-, -T-Ar³- ou -T-Ar³-Q-
où
n représente les nombres 0, 1 ou 2,
Q est l'oxygène ou le soufre,
R⁷ représente l'hydrogène, un groupe cyano, un groupe alkyle, alkoxy, alkylthio, alkylamino ou dialkylamino ayant chacun 1 à 6 atomes de carbone dans les groupes alkyle et chacun étant éventuellement substitué par un radical halogéno, cyano ou alkoxy en C₁ à C₄, ou un groupe cycloalkyle de 3 à 6 atomes de carbone éventuellement substitué par un radical halogéno, cyano, carboxy, alkyle en C₁ à C₄ ou alkoxy en C₁ à C₄, et
R⁸ représente l'hydrogène, un groupe hydroxy, cyano ou un groupe alkyle de 1 à 6 atomes de carbone éventuellement substitué par un radical halogéno, cyano ou alkoxy en C₁ à C₄ ou un groupe cycloalkyle de 3 à 6 atomes de carbone éventuellement substitué par un radical halogéno, cyano, carboxy, alkyle en C₁ à C₄ ou (alkoxy en C₁ à C₄)carbonyle et
Ar³ est un groupe phénylène, naphtylène, cycloalkylène éventuellement substitués chacun une ou plusieurs fois identiques ou différentes ou un groupe hétéroarylène ou hétérocycloalkylène de 3 à 7 atomes dont l'un au moins est un atome d'oxygène, de soufre ou d'azote et le cas échéant un ou deux autres représentent de l'azote, les substituants possibles étant choisis _de préférence dans l'énumération suivante :
halogéno, cyano, nitro, amino, hydroxy, formyle, carboxy, carbamoyle, thiocarbamoyle ;
alkyle, alkoxy, alkylthio, alkylsulfinyle ou alkylsulfonyle chacun linéaire ou ramifié ayant chacun 1 à 6 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéaire ou ramifié et ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximinoalkyle ou alkoximinoalkyle chacun linéaire ou ramifié et ayant chacun 1 à 6 atomes de carbone dans les parties alkyle individuelles ;
cycloalkyle ayant 3 à 6 atomes de carbone et
T représente une liaison simple, l'oxygène, le soufre, un groupe -CH₂-O-, -CH₂-S- ou un groupe alcanediyle ayant 1 à 3 atomes de carbone,
R représente un groupe alkyle, alkoxy, alkylamino, hydroxylamino, alkoxyamino ou dialkylamino ayant chacun 1 à 4 atomes de carbone.

2. Composés de formule (I) suivant la revendication 1, dans laquelle
Ar¹ est un groupe, éventuellement substitué dans chaque cas, ortho-, méta- ou para-phénylène, un groupe furannediyle, thiophènediyle, pyrrolediyle, pyrazolediyle, triazolediyle, oxazolediyle, isoxazolediyle, thiazolediyle, isothiazolediyle, oxadiazolediyle, thiadiazolediyle, pyridinediyle (en particulier pyridine-2,3-diyle), pyrimidinediyle, pyridazinediyle, pyrazinediyle, 1,3,4-triazinediyle ou 1,2,3-triazinediyle, les substituants possibles étant choisis en particulier parmi ceux qui sont énumérés ci-après :
fluor, chlore, cyano, méthyle, éthyle, cyclopropyle, trifluorométhyle, méthoxy, éthoxy, méthylthio, méthylsulfinyle ou méthylsulfonyle,
Ar² représente un groupe phénylène, naphtylène, furannediyle, thiophènediyle, oxazolediyle, isoxazolediyle, thiazolediyle, isothiazolediyle, 1,2,4-oxadiazolediyle, 1,3,4-oxadiazolediyle, 1,2,4-thiadiazolediyle, 1,3,4-thiadiazolediyle, pyridinediyle, pyrimidinediyle, pyridazinediyle, pyrazinediyle, 1,2,3-triazinediyle, 1,2,4-triazinediyle, 1,3,5-triazinediyle, dont chacun porte éventuellement un à trois substituants identiques ou différents, les substituants possibles étant choisis de préférence parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, cyclopropyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle ;
E représente l'un des groupements suivants : 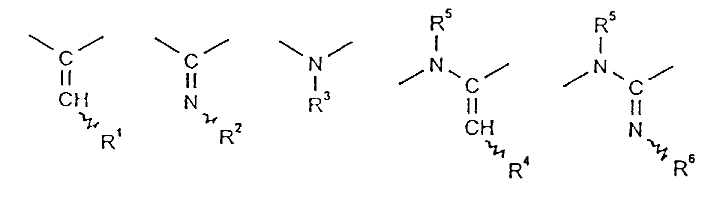 dans lesquels
R¹ est l'hydrogène, le fluor, le chlore, le brome, un groupe cyano ou bien un groupe méthyle, éthyle, propyle, méthoxy, éthoxy, méthylthio, éthylthio, méthylamino, éthylamino ou diméthylamino éventuellement substitué dans chaque cas par du fluor, du chlore, un radical cyano, méthoxy ou éthoxy,
R² représente l'hydrogène, un groupe amino, cyano ou bien un groupe méthyle, éthyle, méthoxy, éthoxy, méthylamino, éthylamino ou diméthylamino dont chacun est éventuellement substitué par du fluor, du chlore, un radical cyano, méthoxy ou éthoxy,
R³ représente l'hydrogène, un groupe cyano ou bien un groupe méthyle, éthyle, n-propyle, isopropyle, n-butyle, isobutyle, sec.-butyle éventuellement substitué dans chaque cas par du fluor, un radical cyano, méthoxy ou éthoxy, un groupe allyle ou propargyle ou un groupe cyclopropyle, cyclobutyle, cyclopentyle, cyclohexyle, cyclopropylméthyle, cyclobutylméthyle, cyclopentylméthyle ou cyclohexylméthyle éventuellement substitué dans chaque cas par du fluor, du chlore, un radical cyano, carboxy, méthyle, éthyle, n-propyle, isopropyle, méthoxycarbonyle ou éthoxycarbonyle,
R⁴ représente l'hydrogène, le fluor, le chlore, le brome, un groupe cyano ou bien un groupe méthyle, éthyle, propyle, méthoxy, éthoxy, méthylthio, éthylthio, méthylamino, éthylamino ou diméthylamino éventuellement substitué dans chaque cas par du fluor, du chlore, un radical cyano, méthoxy ou éthoxy,
R⁵ est un groupe méthyle, éthyle, n-propyle, isopropyle, n-butyle, isobutyle ou sec.-butyle,
R⁶ représente l'hydrogène, un groupe amino, cyano ou bien un groupe méthyle, éthyle, méthoxy, éthoxy, méthylamino, éthylamino ou diméthylamino éventuellement substitué dans chaque cas par du fluor, du chlore, un radical cyano, méthoxy ou éthoxy, et
G représente une liaison simple, l'oxygène, le soufre ou un groupe méthylène, diméthylène (éthane-1,2-diyle), éthène-1,2-diyle, éthyne-1,2-diyle éventuellement substitué par du fluor, du chlore, du brome, un radical hydroxy, méthyle, éthyle, n-propyle, isopropyle, trifluorométhyle, cyclopropyle, cyclobutyle, cyclopentyle ou cyclohexyle, ou l'un des groupements suivants :
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁷)=N-O-, -C(R⁷)=N-O-CH₂-, -N(R⁸)-, -CQ-N(R⁸)-, -N(R⁸)-CQ-, -Q-CQ-N(R⁸)-, -N=C(R⁷)-Q-CH₂-, -CH₂-O-N=C(R⁷)-, -N(R⁸)-CQ-Q-, -CQ-N(R⁸)-CQ-Q-, -N(R⁸)-CQ-Q-CH₂-, -Q-C(R⁷)=N-O-CH₂-, -N(R⁸)-C(R⁷)=N-O-CH₂-, -O-CH₂-C(R⁷)=N-O-, -N=N-C(R⁷)=N-O-,-T-Ar³- ou -T-Ar³-Q-
où
n représente les nombres 0, 1 ou 2,
Q est l'oxygène ou le soufre,
R⁷ représente l'hydrogène, un groupe cyano, un groupe méthyle, éthyle, n-propyle, isopropyle, n-butyle, isobutyle, sec.-butyle, méthoxy, éthoxy, propoxy, butoxy, méthylthio, éthylthio, propylthio, butylthio, méthylamino, éthylamino, propylamino, diméthylamino ou diéthylamino dont chacun est éventuellement substitué par du fluor, du chlore, un radical cyano, méthoxy ou éthoxy, ou bien un groupe cyclopropyle, cyclobutyle, cyclopentyle ou cyclohexyle dont chacun est éventuellement substitué par du fluor, du chlore, un radical cyano, carboxy, méthyle, éthyle, n-propyle, isopropyle, méthoxycarbonyle ou éthoxycarbonyle, et
R⁸ représente l'hydrogène, un groupe hydroxy, cyano ou bien un groupe méthyle, éthyle, n-propyle, isopropyle, n-butyle, isobutyle, sec.-butyle ou tertiobutyle dont chacun est éventuellement substitué par du fluor, du chlore, un radical cyano, méthoxy ou éthoxy, ou un groupe cyclopropyle, cyclobutyle, cyclopentyle ou cyclohexyle dont chacun est éventuellement substitué par du fluor, du chlore, un radical cyano, carboxy, méthyle, éthyle, n-propyle, isopropyle, méthoxycarbonyle ou éthoxycarbonyle,
Ar³ est un groupe phénylène, naphtylène, furannediyle, thiophènediyle, oxazolediyle, isoxazolediyle, thiazolediyle, isothiazolediyle, 1,2,4-oxadiazolediyle, 1,3,4-oxadiazolediyle, 1,2,4-thiadiazolediyle, 1,3,4-thiadiazolediyle, pyridinediyle, pyrimidinediyle, pyridazinediyle, pyrazinediyle, 1,2,3-triazinediyle, 1,2,4-triazinediyle, 1,3,5-triazinediyle, oxirannediyle, oxétanediyle, tétrahydrofurannediyle, perhydropyrannediyle ou pyrrolidinediyle dont chacun porte éventuellement un à trois substituants, les substituants possibles étant choisis de préférence parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle, éthoximinoéthyle ou cyclopropyle et
T représente une liaison simple, l'oxygène, le soufre, un groupe -CH₂-O-, -CH₂-S-, méthylène, éthylène ou propylène et
R représente un groupe méthyle, éthyle, n-propyle, isopropyle, méthoxy, éthoxy, n-propoxy, isopropoxy, méthylamino, éthylamino, n-propylamino, isopropylamino, hydroxylamino, méthoxyamino, diméthylamino, diéthylamino.

3. Composés de formule générale (I) suivant la revendication 1,
dans laquelle
Ar¹ est un groupe orthophénylène, pyridine-2,3-diyle ou thiophène-2,3-diyle,
Ar² est un groupe phénylène éventuellement substitué une à trois fois identiques ou différentes, les substituants possibles étant choisis de préférence parmi ceux qui sont énumérés ci-après :
fluor, chlore, brome, cyano, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorochlorométhoxy, trifluoréthoxy, difluorométhylthio, trifluorométhylthio, difluorochlorométhylthio, trifluorométhylsulfinyle, trifluorométhylsulfonyle, méthoxycarbonyle, éthoxycarbonyle, méthoximinométhyle, éthoximinométhyle, méthoximinoéthyle, éthoximinoéthyle, méthylènedioxy ou éthylènedioxy ayant chacun deux liaisons et chacun étant éventuellement substitué jusqu'à quatre fois identiques ou différentes par du fluor, du chlore, un radical méthyle, trifluorométhyle ou éthyle,
E représente l'un des groupements suivants 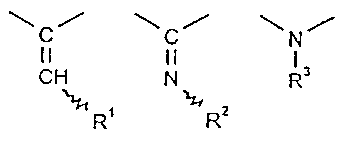 dans lesquels
R¹ et R² représentent dans chaque cas un groupe méthoxy et
R³ est l'hydrogène, un groupe cyano, méthyle, éthyle, n-propyle, isopropyle, méthoxy ou éthoxy,
G représente l'oxygène ou un groupe diméthylène (éthane-1,2-diyle), éthène-1,2-diyle dont chacun est éventuellement substitué par du fluor, du chlore ou du brome, ou l'un des groupements ci-après :
- Q-CQ-, -CQ-Q-, -CH₂-Q-; -Q-CH₂-, -CQ-Q-CH₂-,
-CH₂-Q-CQ-,
-Q-CQ-CH₂-, -Q-CQ-Q-CH₂-, -N=N-, -S(O)ₙ-, -CH₂-S(O)ₙ-,
-CQ-,
-S(O)ₙ-CH₂-, -C(R⁷)=N-O-, -C(R⁷)=N-O-CH₂-, -N(R⁸)-,
-CQ-N(R⁸)-,
-N(R⁸)-CQ-, -Q-CQ-N(R⁸)-, -N=C(R⁷)-Q-CH₂-,
-CH₂-O-N=C(R⁷)-,
-N(R⁸)-CQ-Q-, -CQ-N(R⁸)-CQ-Q-, -N(R⁸)-CQ-Q-CH₂-,
- Q-C(R⁷)=N-O-CH₂-, -N(R⁸)-C(R⁷)=N-O-CH₂-,
-O-CH₂-C(R⁷)=N-O-,
-N=N-C(R⁷)=N-O-, -T-Ar³- ou -T-Ar³-Q-
dans lesquels
n représente les nombres 0, 1 ou 2,
Q est l'oxygène ou le soufre,
R⁷ représente l'hydrogène, un groupe cyano, un groupe méthyle, éthyle ou cyclopropyle et
R⁸ est l'hydrogène, un groupe méthyle, éthyle ou cyclopropyle,
Ar³ représente un groupe phénylène, 1,2,4-thiadiazolediyle, 1,3,4-thiadiazolediyle, 1,2,4-oxadiazolediyle, 1,3,4-oxadiazolediyle, pyridinediyle, pyrimidinediyle, pyridazinediyle, pyrazinediyle, 1,2,3-triazinediyle, 1,2,4-triazinediyle ou 1,3,5-triazinediyle portant chacun le cas échéant un à trois substituants identiques ou différents, les substituants possibles étant choisis de préférence parmi ceux qui sont énumérés ci-après :
fluore, chlore, brome, cyano, méthyle, éthyle, n-propyle, isopropyle, cyclopropyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, trifluorométhylthio, difluorochloro-méthylthio, trifluorométhylsulfinyle ou trifluorométhylsulfonyle et
T représente une liaison simple, l'oxygène, le soufre, un groupe -CH₂-O-, -CH₂-S-, méthylène, éthylène ou propylène et
R est un groupe méthyle, éthyle, méthoxy, méthylamino ou hydroxylamino.

4. Composés de formule (I) suivant la revendication 1,
dans laquelle
Ar¹ est un groupe orthophénylène, pyridine-2,3-diyle ou thiophène-2,3-diyle,
Ar² est un groupe phénylène éventuellement substitué une à trois fois identiques ou différentes, les substituants possibles étant choisis de préférence parmi les substituants énumérés ci-après :
fluor, chlore, brome, cyano, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorochlorométhoxy, trifluoréthoxy, difluorométhylthio, trifluorométhylthio, difluorochlorométhylthio, trifluorométhylsulfinyle ou trifluorométhylsulfonyle, méthoxycarbonyle, éthoxycarbonyle, méthoximinométhyle, éthoximinométhyle, méthoximinoéthyle, éthoximinoéthyle,
méthylènedioxy ou éthylènedioxy ayant chacun deux liaisons et portant chacun le cas échéant un à quatre substituants fluoro, chloro, méthyle, trifluorométhyle ou éthyle identiques ou différents,
E représente l'un des groupements suivants : 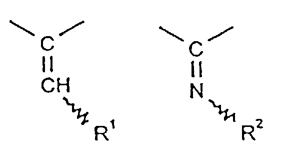 où
R¹ et R² représentent chacun un groupe méthoxy et
G est un groupe -O-CH₂ et
R est un groupe méthyle, éthyle, méthoxy, méthylamino ou hydroxylamino.

5. Composés de formule générale (I) suivant la revendication 1,
dans laquelle
Ar¹ est un groupe orthophénylène, pyridine-2,3-diyle ou thiophène-2,3-diyle,
Ar² représente un groupe phénylène portant le cas échéant un à trois substituants identiques ou différents, les substituants possibles étant choisis de préférence parmi ceux qui sont énumérés ci-après :
fluor, chlore, brome, cyano, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorochlorométhoxy, trifluoréthoxy, difluorométhylthio, trifluorométhylthio, difluorochlorométhylthio, trifluorométhylsulfinyle ou trifluorométhylsulfonyle, méthoxycarbonyle, éthoxycarbonyle, méthoximinométhyle, éthoximinométhyle, méthoximinoéthyle, éthoximinoéthyle,
méthylènedioxy ou éthylènedioxy ayant chacun deux liaisons et chacun étant éventuellement substitué une à quatre fois identiques ou différentes par du fluor, du chlore, un radical méthyle, trifluorométhyle ou éthyle,
E représente l'un des groupements suivants : 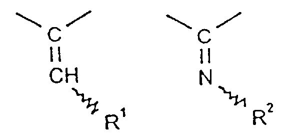 où
R¹ et R² représentent chacun un groupe méthoxy et
G est un groupe -C(R⁷) =N-O-CH₂- dans lequel
R⁷ est l'hydrogène, un groupe cyano, méthyle, éthyle ou cyclopropyle
et
R est un groupe méthyle, éthyle, méthoxy, méthylamino ou hydroxylamino.

6. Composés de formule générale (I) suivant la revendication 1,
dans laquelle
Ar¹ est un groupe orthophénylène,
Ar² est un groupe phénylène éventuellement substitué une à trois fois identiques ou différentes, les substituants possibles étant choisis de préférence parmi ceux qui sont énumérés ci-après :
fluor, chlore, brome, cyano, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorochlorométhoxy, trifluoréthoxy, difluorométhylthio, trifluorométhylthio, difluorochlorométhylthio, trifluorométhylsulfinyle, trifluorométhylsulfonyle, méthoxycarbonyle, éthoxycarbonyle, méthoximinométhyle, éthoximinométhyle, méthoximinoéthyle, éthoximinoéthyle,
méthylènedioxy ou éthylènedioxy ayant chacun deux liaisons et chacun étant éventuellement substitué une à quatre fois identiques ou différentes par du fluor, du chlore, un radical méthyle, trifluorométhyle ou éthyle,
E représente l'un des groupements suivants : 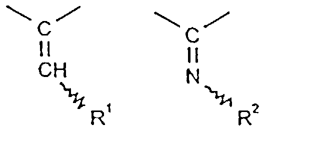 où
R¹ et R² représentent chacun un groupe méthoxy
G est un groupe -T-Ar³-Q- dans lequel
Q xest l'oxygène ou le soufre,
Ar³ est un groupe 1,2,4-thiadiazolediyle, 1,3,4-thiadiazolediyle, 1,2,4-oxadiazolediyle, 1,3,4-oxadiazolediyle, ou un groupe pyridinediyle, pyrimidinediyle ou 1,3,5-triazinediyle portant chacun le cas échéant un ou deux substituants, identiques ou différents, fluor, chlore, cyano, méthyle, cyclopropyle, méthoxy, méthylthio, trifluorométhyle, difluorométhoxy, trifluorométhoxy ou difluorochlorométhoxy,
T est une liaison simple, l'oxygène, le soufre, un groupe -CH₂-O-, -CH₂-S-, méthylène, éthylène ou propylène et
R est un groupe méthyle, éthyle, méthoxy, méthylamino ou hydroxylamino.

7. Compositions pesticides, caractérisées par une teneur en un composé de formule (I) suivant la revendication 1.

8. Procédé pour combattre des parasites, caractérisé en ce qu'on fait agir des composés de formule (I) suivant la revendication 1 sur les parasites et/ou sur leur milieu.

9. Utilisation de composés de formule (I) suivant les revendications 1 à 6 pour combattre des parasites.

10. Procédé de préparation de compositions pesticides, caractérisé en ce qu'on mélange des composés de formule (I) suivant les revendications 1 à 6 avec des diluants et/ou des agents tensioactifs.

11. Procédé de production de composés de formule (I) suivant la revendication 1, caractérisé en ce que
a) on fait réagir des composés hydroxylés de formule générale (II) 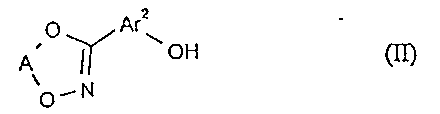 dans laquelle
A et Ar² ont les définitions indiquées dans la revendication 1,
avec des composés halogénés de formule générale (III) 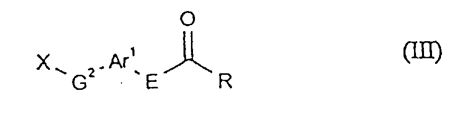 dans laquelle
G² est un groupe -Ar³-Q- ou -Ar³-,
Ar¹, Ar³, E, Q et R ont les définitions indiquées dans la revendication 1 et
X désigne un halogène,
éventuellement en présence d'un accepteur d'acide et en la présence éventuelle d'un diluant ;
b) on fait réagir des composés hydroxylés de formule générale (II) 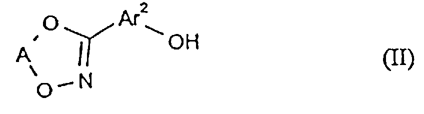 dans laquelle
A et Ar² ont les définitions indiquées dans la revendication 1,
avec des composés halogénés de formule générale (IV) 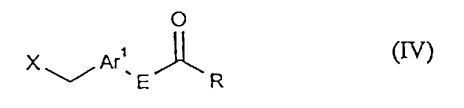 dans laquelle
Ar¹, E et R ont les définitions indiquées dans la revendication 1 et
X représente un halogène,
éventuellement en présence d'un accepteur d'acide et en présence éventuelle d'un diluant ;
c) on fait réagir des esters de formule générale (I-1) 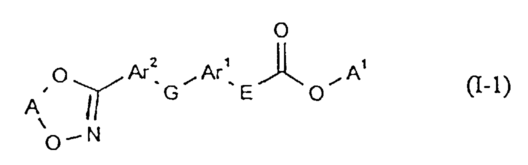 dans laquelle
A, Ar¹, Ar², E et G ont les définitions indiquées dans la revendication 1 et
A¹ est un groupe alkyle,
avec des amines de formule générale (V) 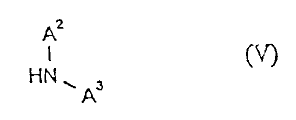 dans laquelle
A² et A³ représentent, indépendamment l'un de l'autre, chacun l'hydrogène ou un groupe alkyle, alkoxy ou hydroxy étant chacun éventuellement substitué,
- ou avec un complexe d'addition d'acide de ces amines -
le cas échéant en présence d'un accepteur d'acide et en présence éventuelle d'un diluant.
